# EUROPEAN PATENT APPLICATION

(11) **EP 4 290 287 A1**
(43) Date of publication of application: **13.12.2023**
(21) Application number: 22749408.5
(22) Date of filing: 07.01.2022
(51) Int. Cl.: G02B 6/42, A61B 90/30, A61J 15/00, A61M 25/095, A61M 39/10, F21S 2/00, F21V 8/00, G02B 6/032, G02B 6/32, G02B 23/26

(54) **ADAPTER**

(30) Priority: 02.02.2021 JP 2021015261; 12.07.2021 JP 2021114764
(71) Applicant: JMS Co., Ltd., Hiroshima-shi Hiroshima 730-8652 (JP); KURUME UNIVERSITY, Kurume-shi Fukuoka 830-0011 (JP); MDI Co., Ltd., Yaizu-shi, Shizuoka 425-0085 (JP)
(72) Inventor: YUKI Takehiko, Hiroshima 730-8652 (JP); MUTSUKADO Masaya, Hiroshima 730-8652 (JP); KANDA Ken, Hiroshima 730-8652 (JP); TOYOTA Koichiro, Hiroshima 730-8652 (JP); KINOSHITA Masahiro, Fukuoka 830-0011 (JP); IWATA Osuke, Fukuoka 830-0011 (JP); MOCHIZUKI Manabu, Shizuoka 425-0085 (JP); IMAMURA Yasuo, Shizuoka 425-0085 (JP)
(74) Representative: Dehns
(86) International application number: PCT/JP2022/000408
(87) International publication number: WO 2022/168529

(57) **Abstract**

A connector (50) includes a tubular member (52) and a base tube (58). A light-guiding tube (60) is drawn out from the base tube (58) toward the opposite side to the tubular member (52). An adapter (1) includes: a first connecting portion (10) that can be connected to and disconnected from a light source device (70); a second connecting portion (20) that can be connected to and disconnected from the connector (50); and a light-guiding member (30) that guides light emitted from the light source device (70) to a proximal end surface (61) of the tube (60).

## Description

### Technical Field

The present invention relates to an adapter for connecting a connector provided at a proximal end of a light-guiding tube to a light source device.

### Background Art

In medical fields, there are cases where a flexible hollow tube is inserted into a patient for examination or treatment. For example, in order to supply a liquid nutrient from the outside to a patient who has difficulty with chewing or swallowing, tube feeding is used in which the nutrient is delivered directly to the stomach via a tube (called an oral tube or a nasal tube, and hereinafter referred to simply as a "tube") inserted through the mouth or nose. It is necessary to confirm that a distal end of the tube is located in the stomach before delivering the nutrient.

Patent Document 1 discloses a method for checking the position of the distal end of a tube by inserting, into the patient, the tube, with an optical fiber inserted therein beforehand, and observing light radiated from the tip of the optical fiber from outside the body. In this method, the optical fiber needs to be pulled out of the tube after the tube has been inserted into the patient and before the nutrient is delivered to the stomach via the tube. Reinserting the optical fiber into the tube after the optical fiber has been pulled out once can cause an accident in which the optical fiber penetrates the tube and damages the digestive tract wall during the reinsertion process. For this reason, there is a problem with the method of Patent Document 1 in that the method can only be used for the initial insertion of the tube into the patient, and cannot be used for subsequent periodic checks of the position of the distal end of the tube.

### Prior Art Documents

### Patent Documents

[Patent Document 1] WO 2015/133119A1
[Patent Document 2] JP 2015-119837A

### Disclosure of Invention

### Problem to be Solved by the Invention

To address the above-described problem, it is conceivable to use the tube itself as a light-guiding path without using an optical fiber. Light is made incident on a proximal end surface of the tube and radiated from the distal end of the tube. With this method, by radiating light from the distal end of the tube, the position of the distal end of the tube can of course be checked when the tube is initially inserted into the patient as well as whenever is necessary thereafter.

For tube feeding, a connector needs to be provided at the proximal end of the tube to form a flow path from a container in which the nutrient is stored to the stomach. Therefore, when light is to be emitted from the distal end of the tube, the connector is connected to a light source device.

However, if the connector is connected directly to the light source device, the connector may be subjected to cross-infection via the light source device. In particular, when a single light source device is used in common for a plurality of patients, the likelihood of cross-infection further increases.

It is conceivable to connect the connector to the light source device via a certain member provided between the connector and the light source device so that the connector does not come into direct contact with the light source device. In this case, it is necessary to ensure that this member does not reduce the amount of light entering the tube.

A first object of the present invention is to make the distal end of a light-guiding tube emit light without cross-infection occurring. A second object of the present invention is to make light from a light source device efficiently enter the light-guiding tube.

### Means for Solving Problem

An adapter of the present invention is an adapter for connecting a connector provided at a proximal end of a flexible hollow light-guiding tube to a light source device. The connector includes a hollow cylindrical tubular member and a hollow cylindrical base tube that is disposed coaxially with the tubular member. The light-guiding tube is drawn out from the base tube toward a side opposite to the tubular member. The adapter includes a first connecting portion that can be connected to and disconnected from the light source device; a second connecting portion that can be connected to and disconnected from the connector; and a light-guiding member that guides light emitted from the light source device to a proximal end surface of the light-guiding tube.

### Effects of the Invention

According to the present invention, the connector is connected to the light source device via the adapter. Therefore, it is possible to make the distal end of the light-guiding tube emit light without cross-infection of the connector occurring.

The adapter includes the light-guiding member that guides light emitted from the light source device to the proximal end surface of the light-guiding tube. Therefore, it is possible to make light from the light source device efficiently enter the light-guiding tube.

### Brief Description of Drawings

[FIG. 1] FIG. 1 shows a schematic configuration of a system for detecting the position of a distal end of a tube, the system employing an adapter according to Embodiment 1 of the present invention.
[FIG. 2A] FIG. 2Ais a perspective view showing the adapter according to Embodiment 1 of the present invention and a connector provided on the tube.
[FIG. 2B] FIG. 2B is a cross-sectional view showing the adapter according to Embodiment 1 of the present invention and the connector provided on the tube.
[FIG. 3] FIG. 3 is a cross-sectional view showing a state in which the connector is connected to a light source device via the adapter according to Embodiment 1 of the present invention.
[FIG. 4A] FIG. 4Ais a perspective view showing an adapter according to Embodiment 2 of the present invention and a connector provided on a tube.
[FIG. 4B] FIG. 4B is a cross-sectional view showing the adapter according to Embodiment 2 of the present invention and the connector provided on the tube.
[FIG. 5] FIG. 5 is a cross-sectional view showing a state in which the connector is connected to a light source device via the adapter according to Embodiment 2 of the present invention.
[FIG. 6A] FIG. 6Ais a perspective view showing an adapter according to Embodiment 3 of the present invention and a connector provided on a tube.
[FIG. 6B] FIG. 6B is a cross-sectional view showing the adapter according to Embodiment 3 of the present invention and the connector provided on the tube.
[FIG. 7] FIG. 7 is a cross-sectional view showing a state in which the connector is connected to a light source device via the adapter according to Embodiment 3 of the present invention.
[FIG. 8] FIG. 8 is a cross-sectional view showing a state in which a connector is connected to a light source device via an adapter according to Embodiment 4 of the present invention.
[FIG. 9A] FIG. 9Ais a perspective view showing an adapter according to Embodiment 5 of the present invention and a connector provided on a tube.
[FIG. 9B] FIG. 9B is a cross-sectional view showing the adapter according to Embodiment 5 of the present invention and the connector provided on the tube.
[FIG. 9C] FIG. 9C is a cross-sectional view showing the adapter according to Embodiment 5 of the present invention and the connector provided on the tube.
[FIG. 10] FIG. 10 is a cross-sectional view showing a state in which the connector is connected to a light source device via the adapter according to Embodiment 5 of the present invention.

### Description of the Invention

In an aspect of the present invention, the light-guiding member may include a light entrance end surface on which light from the light source device is incident. The light entrance end surface may be provided with a convex curved surface. According to this aspect, it is possible to reduce light (leaked light) emerging from a side surface of the light-guiding member. This is advantageous in increasing the brightness of the distal end of the tube.

In an aspect of the present invention, the light-guiding member may include an annular light exit end surface from which light is emitted toward the proximal end surface of the light-guiding tube. According to this aspect, light emerging from the light-guiding member can be made efficiently incident on the proximal end surface of the tube. This is advantageous in increasing the brightness of the distal end of the tube.

In an aspect of the present invention, the light-guiding member may include a light exit end surface from which light is emitted toward the proximal end surface of the light-guiding tube. The light exit end surface may have the function of a lens for focusing light toward the proximal end surface of the light-guiding tube. According to this aspect, light emerging from the light-guiding member can be made efficiently incident on the proximal end surface of the tube. This is advantageous in increasing the brightness of the distal end of the tube.

In an aspect of the present invention, the light-guiding member may include a light entrance end surface on which light from the light source device is incident and a light exit end surface from which light is emitted toward the proximal end surface of the light-guiding tube. The light entrance end surface may have a larger area than the light exit end surface. This aspect is advantageous in increasing the brightness of the distal end of the tube.

In an aspect of the present invention, the light-guiding member may include a light entrance end surface on which light from the light source device is incident and a light exit end surface from which light is emitted toward the proximal end surface of the light-guiding tube. The light-guiding member may have a constant outer diameter between the light entrance end surface and the light exit end surface. According to this aspect, the shape of the light-guiding member is simplified. This is advantageous in reducing the cost of the adapter.

In an aspect of the present invention, when the second connecting portion is connected to the connector, the light-guiding member may oppose the tubular member in the axial direction. According to this aspect, it is possible to simplify the configuration of the adapter.

In an aspect of the present invention, when the second connecting portion is connected to the connector, the light-guiding member may be inserted into the tubular member. According to this aspect, even when the proximal end surface of the light-guiding tube is set back from the leading end of the tubular member, light can be made incident on the proximal end surface of the tube. Thus, irrespective of the inner diameter of the tube, an opening diameter of the leading end of the tubular member can be set to be equal to that of an existing connector. The adapter of the present invention can be applied to a connector that is interchangeable with an existing connector.

In an aspect of the present invention, the adapter may further include a lens for focusing light emitted from the light source device toward a light entrance end surface of the light-guiding member. According to this aspect, light from the light source device can be made efficiently incident on the proximal end surface of the tube. This is advantageous in increasing the brightness of the distal end of the tube.

In an aspect of the present invention, a side surface of the light-guiding member may protrude from a leading end surface of the first connecting portion in the axial direction. According to this aspect, the protruding side surface of the light-guiding member can be used to align the axis of the light-guiding member with the axis of the light source device. This is advantageous in increasing the brightness of the distal end of the tube.

In an aspect of the present invention, the adapter may further include an engagement structure that is engaged with the light source device when the first connecting portion is connected to the light source device. According to this aspect, the adapter can be prevented from dislodging from the light source device. Also, the adapter can be positioned and retained in a predetermined position relative to the light source device in the axial direction.

In an aspect of the present invention, the adapter may further include an intermediate portion between the first connecting portion and the second connecting portion. This aspect is advantageous in reducing the likelihood of the first connecting portion and the second connecting portion being contaminated by the fingers of a user.

In an aspect of the present invention, the intermediate portion may be provided with a holding surface. According to this aspect, the holding surface enables the user to stably hold the adapter and makes it easy for the user to apply a force acting in the axial direction and/or a rotational force around the axis to the adapter.

In an aspect of the present invention, the intermediate portion may be provided with a rising surface that protrudes outward in a radial direction. The rising surface functions as a "holding position restricting structure" that restricts a position at which the intermediate portion is held by the fingers of the user with respect to the axial direction. The rising surface makes it easy to apply a force acting in the axial direction to the adapter. Also, the rising surface reduces the likelihood of the first connecting portion and the second connecting portion being contaminated by the fingers of the user.

In an aspect of the present invention, the second connecting portion may include a fitting structure that can be fitted to the connector. According to this aspect, the connector can be firmly connected to the second connecting portion coaxially therewith. The fitting structure may be, for example, a male tapered surface or a female tapered surface that can establish taper-fitting.

In an aspect of the present invention, the connector may further include an outer cylinder surrounding the tubular member and a female thread provided on an inner circumferential surface of the outer cylinder that opposes the tubular member. According to this aspect, the adapter of the present invention can be applied to a male connector that is provided at a proximal end of a nasal tube used for nasal tube feeding.

Hereinafter, the present invention will be described in detail while showing preferred embodiments thereof. However, it goes without saying that the present invention is not limited to the embodiments below. In the drawings that will be referred to in the following description, only the main members of constituent members of the embodiments of the present invention are shown in a simplified manner for the sake of convenience of description. Accordingly, the present invention may include optional members that are not shown in the drawings below. Moreover, it should be understood that the members shown in the drawings below may be changed or omitted within the scope of the present invention. In the drawings that will be referred to in the description of the embodiments below, members corresponding to those members shown in the drawings that are referred to in the description of any preceding embodiment are denoted by the same reference numerals as the reference numerals of the members shown in the drawings of that preceding embodiment. With respect to such members, redundant descriptions are omitted, and the description of the preceding embodiment should be taken into account as appropriate.

In the present invention, the "axis" of a member (e.g., an adapter, a light-guiding member, or a connector) means the central axis of that member. The "axis" passes through the center of a circle contained in the member and/or coincides with the central axis of a cylinder or cone (taper) contained in the member. Axes are obvious to a person of ordinary skill in the art, and are omitted from the drawings below for the sake of simplicity of the drawings. A direction along a straight line that is orthogonal to an axis is called a "radial direction". With respect to the radial direction, a side nearer to the axis is referred to as an "inner" side, and a side farther from the axis is referred to as an "outer" side. A direction of rotation about the axis is referred to as a "circumferential direction".

### Embodiment 1

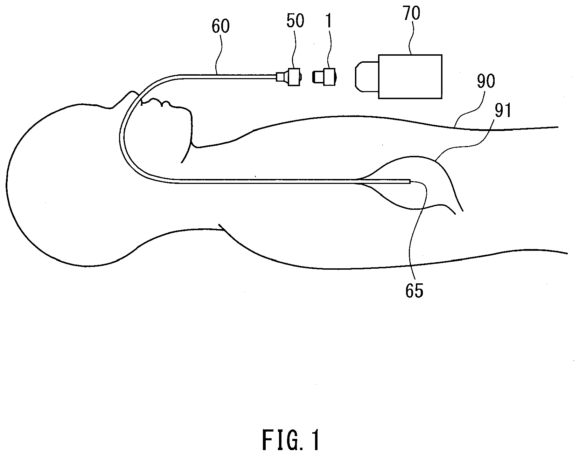

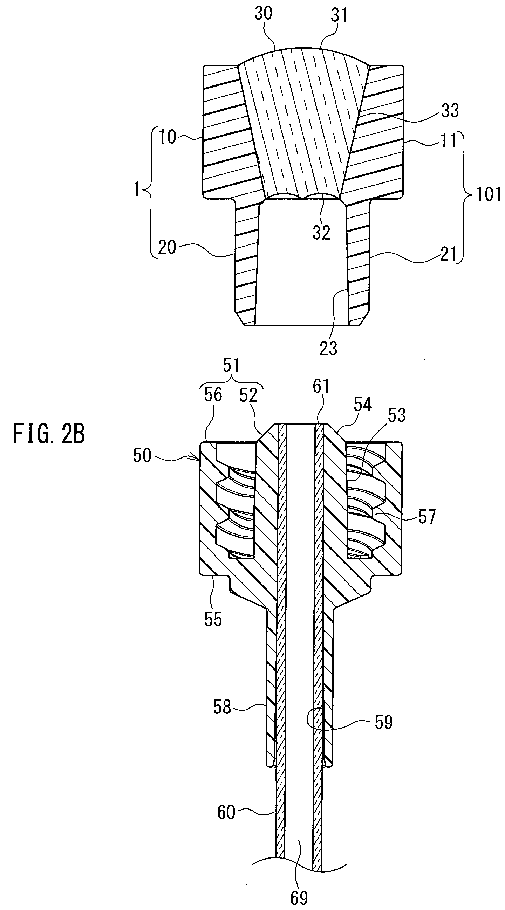

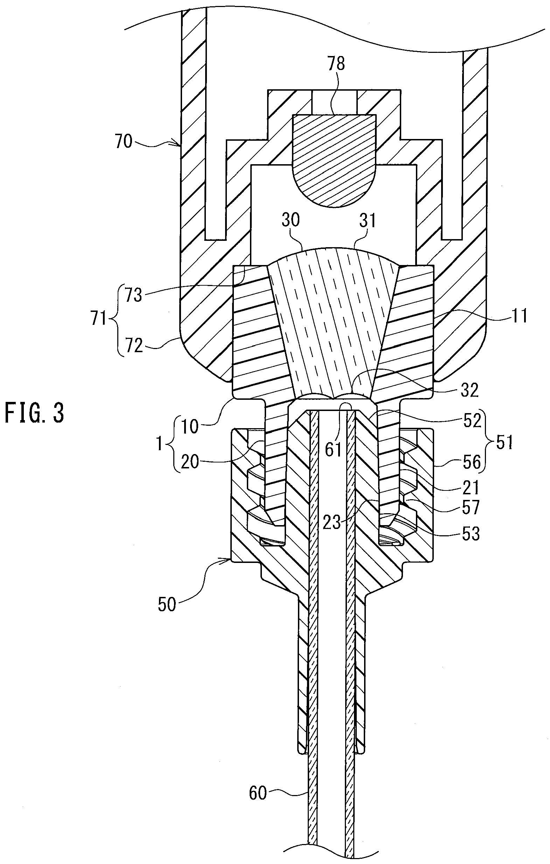

An embodiment in which the present invention is applied to nasal tube feeding will be described. As shown in FIG. 1, a connector 50 is provided at a proximal end of a nasal tube (hereinafter referred to simply as a "tube") 60. The tube 60 is inserted through the nasal cavity of a patient 90, and a distal end 65 of the tube 60 reaches the stomach 91. The tube 60 is flexible so that it can be freely curved and deformed. The tube 60 is a hollow tubular object in which a continuous flow path 69 (see FIGS. 2B, which will be described later) is formed over the entire length thereof. During tube feeding, a liquid nutrient is administered to the stomach 91 of the patient through the connector 50 and the tube 60. The connector 50 can be repeatedly connected to and disconnected from a light source device 70 via an adapter 1 according to Embodiment 1 of the present invention. When the connector 50 is connected to the light source device 70 via the adapter 1, light from a light source 78 (see FIG. 3, which will be described later) incorporated in the light source device 70 sequentially passes through the adapter 1, the connector 50, and the tube 60, and emerges from the distal end 65 of the tube 60. The light from the distal end 65 is transmitted through the body of the patient 90 and causes the body surface to glow An operator can check the position of the distal end 65 of the tube 60 based on the light emission position on the body surface of the patient 90.

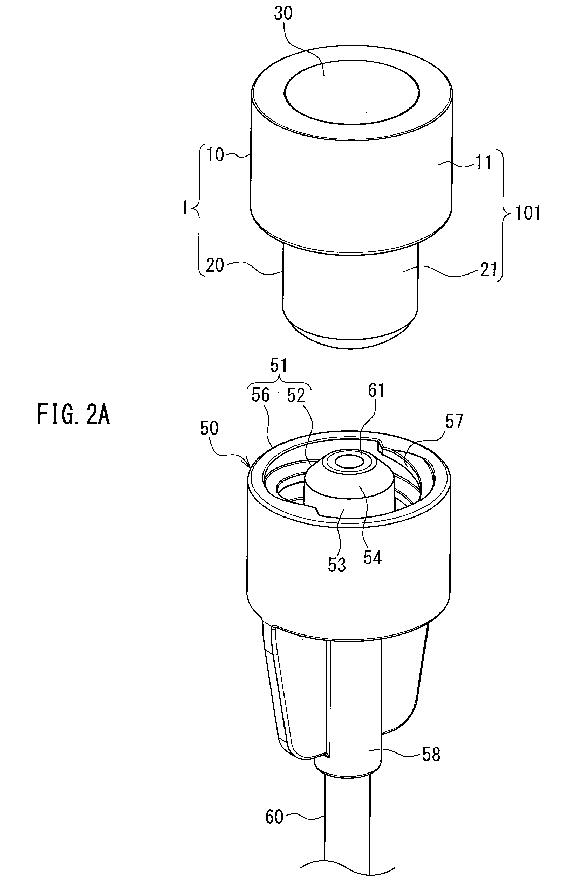

FIG. 2Ais a perspective view showing the adapter 1 and the connector 50 provided on the tube 60. FIG. 2B is a cross-sectional view taken along a plane containing the axes (not shown) of the adapter 1 and the connector 50 in FIG. 2A

The adapter 1 includes a first connecting portion 10 that can be connected to and disconnected from the light source device 70 (see FIG. 1) at one end and a second connecting portion 20 that can be connected to and disconnected from the connector 50 at the other end. The first connecting portion 10 and the second connecting portion 20 are arranged coaxially. The first connecting portion 10 includes a hollow cylindrical first connecting tube 11. An outer circumferential surface of the first connecting tube 11 is a cylindrical surface whose outer diameter is constant with respect to the axial direction. However, the outer circumferential surface of the first connecting tube 11 is not limited to this configuration, and may also be, for example, a tapered surface whose outer diameter gradually increases toward a leading end side or a base end side (second connecting portion 20 side) of the first connecting tube 11. The second connecting portion 20 includes a hollow cylindrical second connecting tube 21. An inner circumferential surface of the second connecting tube 21 has a female tapered surface 23 whose inner diameter gradually increases toward a leading end thereof (an end opposite to the first connecting portion 10).

Alight-guiding member 30 is housed in the first connecting tube 11. The light-guiding member 30 includes a light entrance end surface 31 and a light exit end surface 32 that face opposite sides to each other in the axial direction, as well as a side surface 33 connecting the light entrance end surface 31 and the light exit end surface 32 to each other. The light-guiding member 30 has a substantially truncated cone shape that is larger in diameter on the light entrance end surface 31 side. The light-guiding member 30 is fitted into the first connecting tube 11, with the conical or tapered side surface 33 being in surface contact with the inner circumferential surface of the first connecting tube 11 while leaving substantially no space therebetween. The light entrance end surface 31 faces the leading end side of the first connecting portion 10 (the opposite side to the second connecting portion 20), and is a convex curved surface in Embodiment 1. The light exit end surface 32 faces an inner cavity of the second connecting tube 21, and is an annular groove extending continuously around the axis of the adapter 1 in Embodiment 1. More specifically, as shown in FIG. 2B, a cross-sectional shape of the light exit end surface 32 taken along a plane containing the axis of the adapter 1 includes two substantially circular arcs that are symmetrical with respect to the axis. The light exit end surface 32 is an annular concave curved surface formed according to a trajectory that is obtained when these two substantially circular arcs rotate around the axis.

The adapter 1 is composed of two components: the light-guiding member 30 and a portion (adapter main body 101) other than the light-guiding member 30. The adapter main body 101 is made of a material that has such a mechanical strength (stiffness) that it will not be substantially deformed by an external force. As such a material, for example, resin materials such as polypropylene, acrylonitrile-butadiene-styrene copolymers, polycarbonate, polyacetal, polystyrene, polyamide, polyethylene, hard polyvinyl chloride, and the like can be used. The entire adapter main body 101 can be integrally formed as a single component using an aforementioned resin material.

The light-guiding member 30 is made of a material (light-guiding material) having light-guiding properties so that light can enter the light-guiding member 30 from the light entrance end surface 31, pass through the light-guiding member 30, and emerge from the light exit end surface 32. As such a light-guiding material, transparent materials such as, for example, resin materials (polypropylene, acrylonitrile-butadiene-styrene copolymers, hard polyvinyl chloride, polyurethane, silicone, polyethylene, styrene elastomers, polybutadiene, polyolefin, acrylic resins (e.g., PMMA), polycarbonate, and the like) and quartz glass can be used. Of these, a resin material is preferable in view of its moldability and handleability, and an acrylic resin (e.g., PMMA) or polycarbonate is more preferable. In order to increase the brightness (luminous flux) of the distal end 65 (see FIG. 1) of the tube 60, it is preferable to reduce light loss between the light entrance end surface 31 and the light exit end surface 32. For this purpose, it is effective to reduce light (leaked light) emerging from the side surface 33. Although there is no limitation on the means for reducing leaked light, for example, the following techniques can be used: (1) configuring the light-guiding member 30 as a light-guiding member with a multilayer structure including at least an inner layer having a high refractive index and an outer layer having a low refractive index; (2) providing a reflective layer (e.g., a metal vapor deposition layer made of silver, aluminum, or the like) on the side surface 33; and so on.

There is no limitation on the method for integrating the light-guiding member 30 into the adapter main body 101. For example, a method in which the light-guiding member 30 and the adapter main body 101 are produced separately and then the light-guiding member 30 is fitted into the first connecting tube 11 of the adapter main body 101; a method in which one of the light-guiding member 30 and the adapter main body 101 is produced and then the other is integrated into the produced light-guiding member 30 or adapter main body 101 through double molding; and other methods can be used.

The connector 50 includes a connector portion 51 on a leading end side (adapter 1 side) thereof, and a base tube 58 on a base end side thereof. The connector portion 51 has a tubular member 52 with a hollow tubular shape and an outer cylinder 56 surrounding the tubular member 52. The connector portion 51 (or the tubular member 52) and the base tube 58 are arranged coaxially. A through hole 59 extends through the connector 50 from the tubular member 52 to the base tube 58 along the axis of the connector 50. An inner circumferential surface of the through hole 59 is a cylindrical surface that is coaxial with the axis of the connector 50.

An outer circumferential surface of the tubular member 52 is provided with a male tapered surface 53 whose outer diameter gradually decreases toward a leading end of the tubular member 52. The outer circumferential surface of the tubular member 52 further has, on a side nearer to the leading end thereof than the male tapered surface 53, a leading end tapered surface 54 having a larger taper angle than the male tapered surface 53. The leading end (a portion or the entirety of the leading end tapered surface 54) of the tubular member 52 protrudes from a leading end of the outer cylinder 56 in the axial direction of the connector 50. The configuration in which the tubular member 52 protrudes from the outer cylinder 56 is advantageous in facilitating the connection of the connector 50 (or the tubular member 52) to the adapter 1.

A flange 55 protrudes outward in the radial direction from a base end of the tubular member 52. The outer cylinder 56 extends from a circular outer circumferential edge of the flange 55 toward the same side as the tubular member 52 (toward the leading end side of the connector 50). The outer cylinder 56 has a substantially cylindrical shape and is disposed coaxially with the tubular member 52. The outer cylinder 56 is spaced apart from the tubular member 52 in the radial direction. A female thread 57 is provided on an inner circumferential surface of the outer cylinder 56 that opposes the tubular member 52.

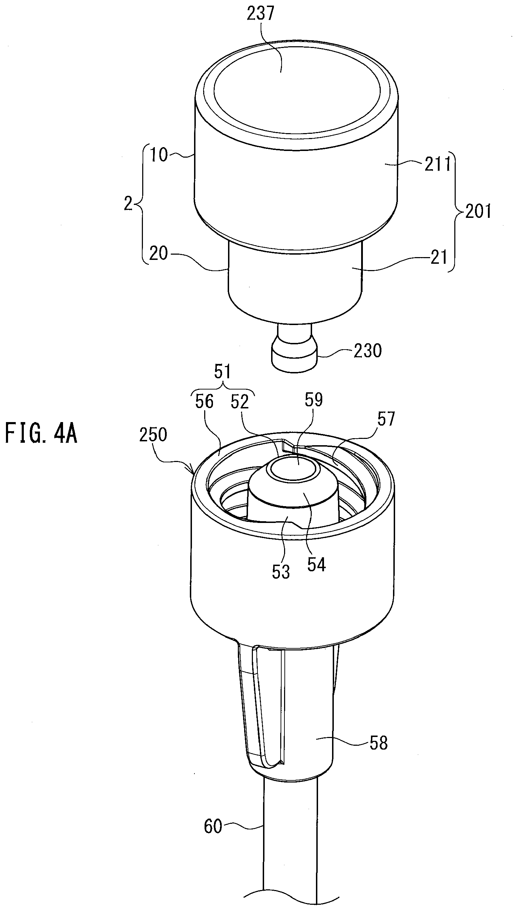

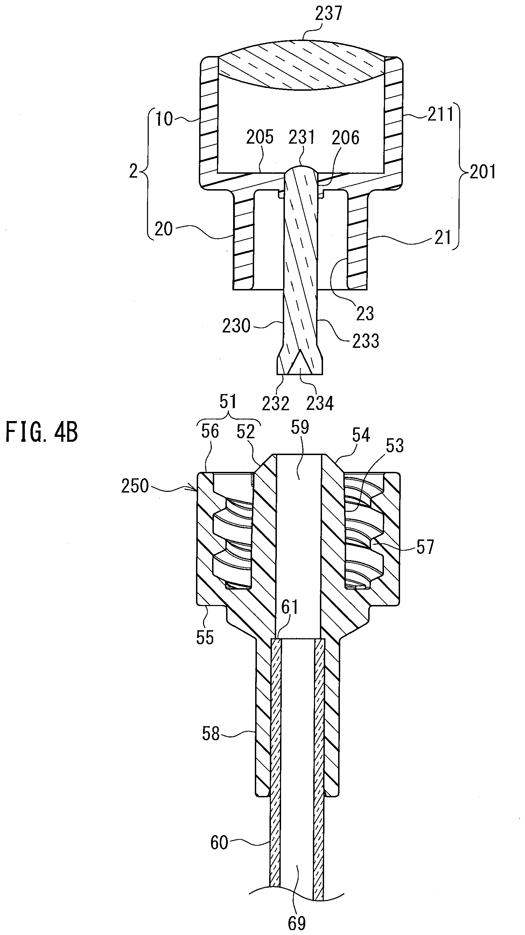

The connector portion 51 is configured to be interchangeable with a male connector (see FIGS. 4A and 4B of Patent Document 2, for example) that is used for tube feeding.

The flexible hollow tube 60 is inserted into the through hole 59 of the connector 50. The outer diameter of the tube 60 is substantially equal to the inner diameter of the through hole 59. The tube 60 has a proximal end surface 61, which is a terminal end surface on the proximal end side. The proximal end surface 61 is a flat surface that is perpendicular to the longitudinal direction of the tube 60. The proximal end surface 61 is flush with the leading end of the tubular member 52 and is exposed to the outside. The tube 60 is fixed to the connector 50 (in particular, the base tube 58) using an adhesive or the like so as not to separate from the connector 50. The tube 60 is drawn out from the base tube 58 toward the opposite side to the tubular member 52.

The connector 50 is made of a material that has such a mechanical strength (stiffness) that it will not be substantially deformed by an external force. As such a material, the resin materials listed above as examples of the material of the adapter main body 101 of the adapter 1 can be used. The entire connector 50 can be integrally formed as a single component using an aforementioned resin material.

Although there is no limitation on the material of the tube 60, a material having flexibility and light-guiding properties is preferable, and, for example, resins such as polyurethane, acrylic, silicone, polyethylene, styrene elastomers, polybutadiene, and polyolefin can be used. In the present embodiment, light entering from the proximal end surface 61 of the tube 60 is transmitted through the tube 60 (a portion between an inner circumferential surface and an outer circumferential surface of the tube 60, the portion constituting the thickness of the tube 60) and emerges from the distal end 65 (see FIG. 1), and thus, the distal end 65 emits light. In order to increase the brightness (luminous flux) of the distal end 65, it is preferable to reduce light loss between the proximal end surface 61 and the distal end 65. For this purpose, it is effective to reduce light (leaked light) emerging to the outside from the outer surface of the tube 60 between the proximal end surface 61 and the distal end 65. Although there is no limitation on the means for reducing leaked light, for example, one, or a combination of two or more, of the following techniques can be used: (1) smoothing the outer circumferential surface of the tube 60; (2) covering the outer circumferential surface of the tube 60 with a coating material having a lower refractive index than the tube 60; (3) providing a reflective layer (e.g., a metal vapor deposition layer made of silver, aluminum, or the like) on the outer circumferential surface of the tube 60; (4) configuring the tube 60 as a tube with a multilayer structure including at least an inner layer having a high refractive index and an outer layer having a low refractive index; and so on.

In order to ensure that the light from the distal end 65 causes the body surface of the patient 90 to glow irrespective of the orientation of the distal end 65 in the stomach 91 (see FIG. 1), it is preferable that light emerging from the distal end 65 of the tube 60 emerges radially in a direction perpendicular or oblique to the longitudinal direction of the tube 60. To achieve this, preferably, for example, an inclined light exit surface is provided at the distal end 65 of the tube 60, and/or a reflective member or a refractive member is provided opposing a light exit surface at the distal end 65 of the tube 60.

As shown in FIG. 3, the adapter 1 is used to connect the connector 50 to the light source device 70.

The light source device 70 includes a retaining portion 71 in which the first connecting portion 10 (first connecting tube 11) is accommodated and retained, and the light source 78 disposed at a position farther inward than the retaining portion 71. The retaining portion 71 includes a retaining cylinder 72 and a step surface 73. An inner circumferential surface of the retaining cylinder 72 is a cylindrical surface having substantially the same diameter as the first connecting tube 11. The step surface 73 is formed at a position located at a predetermined distance from an opening end of the retaining cylinder 72 and protrudes inward in the radial direction from the inner circumferential surface of the retaining cylinder 72. The light source 78 is disposed coaxially with the retaining portion 71 (or the retaining cylinder 72). The first connecting portion 10 of the adapter 1 is inserted into the retaining portion 71 until the leading end of the first connecting tube 11 abuts against the step surface 73. The retaining cylinder 72 positions the first connecting portion 10 (or the light-guiding member 30) coaxially with the light source 78. The step surface 73 positions the first connecting portion 10 (or the light-guiding member 30) at a position located at a predetermined distance from the light source 78 in the axial direction. The light entrance end surface 31 of the light-guiding member 30 opposes the light source 78.

The second connecting portion 20 (or the second connecting tube 21) of the adapter 1 is inserted into a gap between the outer cylinder 56 and the tubular member 52 of the connector 50. The tubular member 52 of the connector 50 is inserted into the second connecting portion 20 (or the second connecting tube 21). The female tapered surface 23 of the second connecting tube 21 matches the male tapered surface 53 of the tubular member 52 in terms of the outer diameter and the taper angle. Thus, the male tapered surface 53 is taper-fitted to the female tapered surface 23. The tubular member 52 (or the proximal end surface 61 of the tube 60) is positioned coaxially with the light-guiding member 30 and at a position located a predetermined distance away from the light-guiding member 30 in the axial direction. The proximal end surface 61 of the tube 60 opposes the light exit end surface 32 of the light-guiding member 30.

In this state, the light source 78 is made to emit light. The light source 78 emits light toward the light entrance end surface 31 of the light-guiding member 30. The light emitted from the light source 78 is incident on the light entrance end surface 31, passes through the light-guiding member 30, emerges from the light exit end surface 32, and is then incident on the proximal end surface 61 of the tube 60.

Although there is no limitation on the light source 78, a light emitting diode (LED) can be used. It is preferable that the light emitted by the light source 78 is visible light or near-infrared light. Although there is no limitation on the wavelength of the light, it is preferable that the wavelength of the light is 360 nm or more, and more preferably 630 nm or more, and is 3,000 nm or less, and more preferably 780 nm or less. Light having a wavelength within this range has a high transmittance through the human body, and is also minimally invasive to the human body and hence highly safe. Since visible light can be observed with the naked eye, the position of the distal end 65 of the tube 60 can be easily checked. Near-infrared light is more penetrating than visible light and can be observed via a dedicated camera such as an infrared camera. In one example, light having a wavelength of 630 nm can be used as visible light, or light having a wavelength of 780 nm can be used as near-infrared light. The light source 78 may include a lens and/or a lens may be provided between the light source 78 and the light-guiding member 30 so that the light emitted from the light source 78 is efficiently incident on the light entrance end surface 31 of the light-guiding member 30.

A method of using the adapter 1 of Embodiment 1 will be described.

A nasal tube in which the connector 50 is provided at the proximal end of the tube 60 is prepared. Like a common nasal tube, the tube 60 is inserted into the nasal cavity of the patient 90 (see FIG. 1). The adapter 1 is connected to the connector 50, and the adapter 1 is connected to the light source device 70. Then, the light source 78 (see FIG. 3) is made to emit light. The light emitted from the light source 78 sequentially passes through the light-guiding member 30 and the tube 60 and is radiated from the distal end 65 of the tube 60. The light from the distal end 65 is transmitted through the patient 90. An operator can check the position of the distal end 65 based on the light emission position on the body surface of the patient 90. Light can be seen with the naked eye or via an infrared camera, depending on its wavelength.

As is the case with when inserting a common nasal tube, the tube 60 with a stylet (also called a guide wire) inserted into its flow path 69 beforehand may also be inserted into the patient 90. The proximal end of the stylet can be drawn out from the connector 50. In this case, the stylet is pulled out of the tube 60 before the connector 50 is connected to the light source device 1.

Also, the tube 60 with an optical fiber inserted into its flow path 69 such that the tip of the optical fiber reaches the distal end 65 of the tube 60 may be inserted into the patient 90. In addition to the distal end 65, the tip of the optical fiber is also made to emit light using the light source device 70. Thus, the luminous flux from the distal end 65 increases, and therefore the position of the distal end 65 can be checked with higher accuracy. The optical fiber is pulled out of the tube 60 after it is confirmed that the distal end of the tube 60 has reached the stomach.

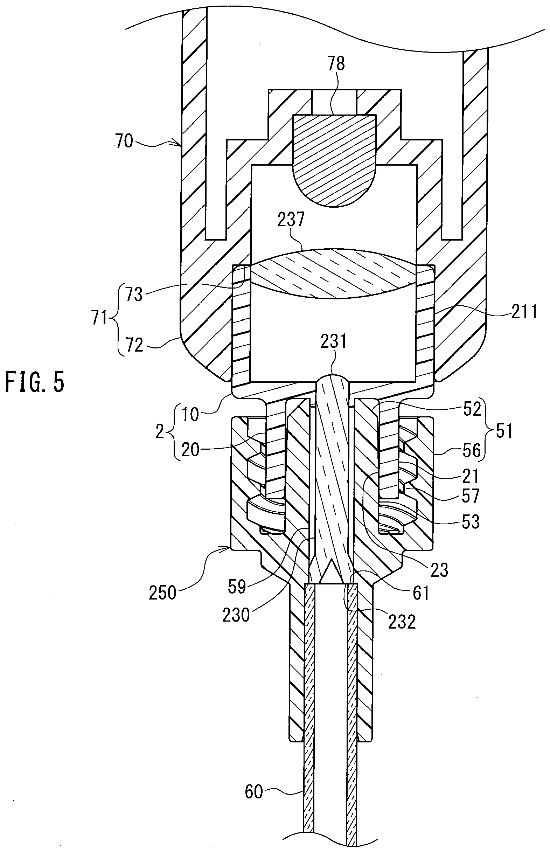

The adapter 1 is disconnected from the connector 50 after it is confirmed that the distal end 65 of the tube 60 has reached the stomach. Then, the connector 50 is connected to a connector (a female connector, see FIGS. 5A and 5B of Patent Document 2, for example) provided at a downstream end of a tube for conveying the nutrient (generally called a tube feeding set). The nutrient flows through the flow path 69 of the tube 60 and is discharged from the distal end 65 and administered to the patient.

The connector 50 and the tube 60 are left with the patient 90 for several days. During this period, the distal end 65 may move due to the tube 60 curling up, for example. To address this issue, the connector 50 is connected to the light source device 70 via the adapter 1 to make the distal end 65 emit light and thereby enable checking of the position of the distal end 65, at predetermined time intervals (e.g., immediately before administration of a nutrient to the patient 90).

As described above, the adapter 1 of Embodiment 1 includes the first connecting portion 10 that can be connected to and disconnected from the light source device 70 and the second connecting portion 20 that can be connected to and disconnected from the connector 50. Thus, the connector 50 can be connected to the light source device 70 via the adapter 1. The adapter 1 further includes the light-guiding member 30 that guides light emitted from the light source device 70 to the proximal end surface 61 of the tube 60. Therefore, when light from the light source 78 is made incident on the light entrance end surface 31 of the light-guiding member 30, the light passes through the light-guiding member 30, emerges from the light exit end surface 32, and can then be incident on the proximal end surface 61 of the tube 60. The light further passes through the tube 60 and emerges from the distal end 65 of the tube 60. According to Embodiment 1, unlike Patent Document 1, it is possible to make the distal end 65 of the tube 60 emit light by using the tube 60 itself as a light-guiding path. The light from the distal end 65 can be observed through the body of the patient 90. Thus, it is possible to easily and accurately detect the position of the distal end 65 of the tube 60.

In Patent Document 1 above, it is necessary to insert an optical fiber into the tube in order to check the position of the distal end of the tube. Reinserting the optical fiber into the tube after the optical fiber has been pulled out of the tube can cause an accident in which the optical fiber penetrates the tube and damages the digestive tract wall. In contrast, in Embodiment 1, the optical fiber, which is essential in Patent Document 1, is not necessary. The position of the distal end 65 of the tube 60 can be checked without the need to reinsert the optical fiber into the tube 60. Therefore, in Embodiment 1, the aforementioned accident that may occur in Patent Document 1 can be avoided. After the tube 60 has been inserted into the patient 90, the position of the distal end 65 of the tube 60 can be safely checked whenever is necessary, by radiating light from the distal end 65 of the tube 60.

The connector 50 is connected to the light source device 70 via the adapter 1. The connector 50 can be connected to the light source device 70 via the adapter 1 even when the position of the distal end 65 needs to be checked after the tube 60 has been inserted into the patient 90, not to mention when the tube 60 is initially inserted into the patient. There is no need to directly connect the connector 50 to the light source device 70 in order to check the position of the distal end 65. Even if the light source device 70 (in particular, the retaining portion 71) is contaminated, the connector 50 is unlikely to be contaminated if a clean adapter 1 is used. For example, even when a single light source device 70 is used in common for a plurality of patients, the occurrence of cross-infection can be prevented by using a separate clean adapter 1 for each patient. Therefore, according to Embodiment 1, it is possible to make the distal end 65 of the tube 60 emit light without cross-infection occurring. Preferably, the adapter 1 is cleaned and sterilized after each use. More preferably, the adapter 1 is discarded after each use, and a new adapter 1 is always used.

The adapter 1 includes the light-guiding member 30 that guides light emitted from the light source 78 to the proximal end surface 61 of the tube 60. Thus, light from the light source 78 can be made to efficiently enter the tube 60. This is advantageous in increasing the brightness (luminous flux) of the distal end 65 of the tube 60.

The light-guiding member 30 includes the light entrance end surface 31 provided with a convex curved surface. The convex curved surface refracts light from the light source 78 such that the light converges on the axis of the light-guiding member 30. This is advantageous in reducing light (leaked light) emerging from the side surface 33 of the light-guiding member 30. However, in the present invention, the light entrance end surface 31 need not have a convex curved surface. The light entrance end surface 31 may be a flat surface that is perpendicular to the axis of the adapter 1, or may be a concave curved surface.

The light-guiding member 30 includes the light exit end surface 32 that has an annular shape and opposes the proximal end surface 61, which has an annular shape, of the tube 60 in the axial direction. The annular light exit end surface 32 is advantageous in enabling light emerging from the light-guiding member 30 to be incident to the full extent on the proximal end surface 61 of the tube 60.

An annular concave curved surface provided at the light exit end surface 32 has the function of a lens that refracts light emerging from the light-guiding member 30 such that the light converges on the annular proximal end surface 61. This is further advantageous in enabling light emerging from the light-guiding member 30 to be efficiently incident on the proximal end surface 61 of the tube 60.

However, in the present invention, the shape of the light exit end surface 32 is not limited to the aforementioned shape. For example, the light exit end surface 32 may be a non-annular concave curved surface, or a flat surface that is perpendicular to the axis of the adapter 1. The flat surface may be an annular flat surface like a light exit end surface 232 (see FIG. 4B) of Embodiment 2, which will be described later. Alternatively, the light exit end surface 32 may be a convex curved surface. The convex curved surface 32 may be an annular convex curved surface. The cross-sectional shape of the annular convex curved surface taken along a plane containing the axis of the adapter 1 includes two substantially circular arcs that are symmetrical to each other with respect to the axis, and the annular convex curved surface may be formed according to a trajectory that is obtained when the two substantially circular arcs are rotated around the axis.

When the second connecting portion 20 is connected to the connector 50, the light-guiding member 30 is not inserted into the tubular member 52, but opposes the leading end of the tubular member 52 in the axial direction. This is advantageous in simplifying the configuration of the adapter 1. Thus, it is possible to easily optically couple the light-guiding member 30 to the tube 60 using a simple and inexpensive adapter 1.

The light-guiding member 30 has a substantially truncated cone shape in which the light entrance end surface 31 is larger in diameter than the light exit end surface 32. A larger amount of light can enter the light-guiding member 30 via the light entrance end surface 31 that has a large area. This is advantageous in increasing the brightness (luminous flux) of the distal end 65 of the tube 60.
However, the present invention is not limited to this configuration. For example, the light-guiding member 30 may have a substantially cylindrical column shape, with the side surface 33 being a cylindrical surface having a constant outer diameter with respect to the axial direction.

In Embodiment 1, the outer circumferential surface of the second connecting tube 21 may be provided with a male thread that can be screwed to the female thread 57 of the connector 50.

The second connecting portion 20 may be configured such that the outer cylinder 56, rather than the tubular member 52, of the connector 50 is fitted into the second connecting tube 21. In this case, the inner circumferential surface 23 of the second connecting tube 21 is configured to be fitted to the outer circumferential surface of the outer cylinder 56.

In Embodiment 1, the proximal end surface 61 of the tube 60 need not be located at exactly the same position as the leading end of the tubular member 52 in the axial direction of the connector 50. For example, the proximal end surface 61 may be slightly set back or protruding from the leading end of the tubular member 52. Even in this case, light emerging from the light exit end surface 32 of the light-guiding member 30 can be made incident on the proximal end surface 61 of the tube 60.

### Embodiment 2

FIG. 4Ais a perspective view showing an adapter 2 according to Embodiment 2 of the present invention and a connector 250 provided at the proximal end of the tube 60. FIG. 4B is a cross-sectional view taken along a plane containing the axes (not shown) of the adapter 2 and the connector 250 in FIG. 4A. The adapter 2 of Embodiment 2 will be described focusing primarily on the differences from Embodiment 1.

The adapter 2 includes the first connecting portion 10 at one end and the second connecting portion 20 at the other end. The first connecting portion 10 and the second connecting portion 20 are arranged coaxially. The first connecting portion 10 includes a hollow cylindrical first connecting tube 211. An outer circumferential surface of the first connecting tube 211 is a cylindrical surface whose outer diameter is constant with respect to the axial direction, as in the case of the first connecting tube 11 of Embodiment 1. However, the outer circumferential surface of the first connecting tube 211 is not limited to this configuration, and may also be, for example, a tapered surface whose outer diameter gradually increases toward a leading end side or a base end side (second connecting portion 20 side) of the first connecting tube 211. A lens 237 is provided at or near the leading end (the end opposite to the second connecting portion 20) of the first connecting tube 211 so as to block an opening of the first connecting tube 211 and to be coaxial with the first connecting tube 211.

The adapter 2 further includes a partition wall 205 that separates an inner cavity of the first connecting portion 211 and an inner cavity of the second connecting tube 21 from each other. The partition wall 205 may be a flat plate that is perpendicular to the axis of the adapter 2. The partition wall 205 has a hole 206 at the center, the hole 206 penetrating the partition wall 205 in the axial direction. The light-guiding member 230 is retained by the partition wall 205 such that it blocks the hole 206. The light-guiding member 230 has an elongated rod shape (substantially cylindrical column shape) and extends coaxially with the adapter 2. The light-guiding member 230 includes a light entrance end surface 231 and a light exit end surface 232 that face opposite sides to each other in the axial direction. The light entrance end surface 231 faces the leading end side of the first connecting portion 10 (the opposite side to the second connecting portion 20), and is a convex curved surface in Embodiment 2. The light exit end surface 232 faces the opposite side to the first connecting portion 10, and is a flat surface that is perpendicular to the axis of the adapter 2. More specifically, the light exit end surface 232 is an annular flat surface surrounding a conical recess 234 that is coaxial with the adapter 2. Aside surface (outer circumferential surface) 233 of the light-guiding member 230 slightly increases in diameter near the light exit end surface 232. The light-guiding member 230 protrudes further than the second connecting tube 21 of the second connecting portion 20 in the axial direction. The second connecting tube 21 surrounds the light-guiding member 230 while being spaced apart from the light-guiding member 230 in the radial direction.

The adapter 2 is composed of three components: the light-guiding member 230, the lens 237, and a portion (an adapter main body 201) other than the light-guiding member 230 and the lens 237. The adapter main body 201 is made of a material that has such a mechanical strength (stiffness) that it will not be substantially deformed by an external force. Materials that can be used for the adapter main body 201 of Embodiment 1 can be used as the material of the adapter main body 201 of Embodiment 2.

The light-guiding member 230 is made of a material (light-guiding material) having light-guiding properties so that light can enter the light-guiding member 230 from the light entrance end surface 231, pass through the light-guiding member 230, and emerge from the light exit end surface 232. The lens 237 is made of a material (light-guiding material) having light-guiding properties so that light emitted from the light source 78 (see FIG. 5, which will be described later) can be focused onto the light entrance end surface 231 of the light-guiding member 230. Light-guiding materials that can be used for the light-guiding member 30 of Embodiment 1 can be used as the light-guiding materials of the light-guiding member 230 and the lens 237. In addition, the means for reducing leaked light from the side surface 33 of the light-guiding member 30, which has been described in Embodiment 1, can also be applied to the light-guiding member 230 as well.

There is no limitation on the method for integrating the light-guiding member 230 and the lens 237 into the adapter main body 201. For example, a method in which the light-guiding member 230, the lens 237, and the adapter main body 201 are produced separately, then the light-guiding member 230 is fitted into the hole 206 of the partition wall 205, and the lens 237 is fitted into the first connecting tube 211; a method in which one of the light-guiding member 230 and the lens 237, as well as the adapter main body 201 are integrally produced through double molding, and then the other of the light-guiding member 230 and the lens 237 is fitted into the adapter main body 201; and other methods can be used.

The connector 250 and the tube 60 are substantially the same as the connector 50 and the tube 60 (see FIGS. 2A and 2B) of Embodiment 1. However, in Embodiment 2, the proximal end surface 61 of the tube 60 is located at a position that is set back from the leading end of the tubular member 52. The proximal end surface 61 faces the leading end side of the tubular member 52. In the present embodiment,, the tube 60 is not substantially inserted into the tubular member 52. However, the present invention is not limited to this configuration, and the tube 60 may extend into the tubular member 52 (that is, the proximal end surface 61 may be located within the tubular member 52).

FIG. 5 is a cross-sectional view showing a state in which the connector 250 is connected to the light source device 70 via the adapter 2. The light source device 70 is the same as that of Embodiment 1. The first connecting portion 10 of the adapter 2 is inserted into the retaining portion 71 until the leading end of the first connecting tube 211 abuts against the step surface 73. The retaining cylinder 72 positions the first connecting portion 10 (or the lens 237) coaxially with the light source 78. The step surface 73 positions the first connecting portion 10 (or the lens 237) at a position located at a predetermined distance from the light source 78 in the axial direction. The lens 237 opposes the light source 78.

The second connecting portion 20 (or the second connecting tube 21) of the adapter 2 is connected to the connector 250 in a similar manner to that of Embodiment 1. The male tapered surface 53 of the tubular member 52 is taper-fitted to the female tapered surface 23 of the second connecting tube 21. In Embodiment 2, the light-guiding member 230 is inserted into the through hole 59 of the tubular member 52. The light-guiding member 230 is positioned coaxially with the tube 60. The light exit end surface 232 of the light-guiding member 230 opposes the proximal end surface 61 of the tube 60 in the axial direction and is located in close proximity to, or preferably abuts against, the proximal end surface 61.

The light source 78 emits light toward the lens 237. The lens 237 focuses light emitted from the light source 78, toward the light entrance end surface 231 of the light-guiding member 230. Light incident on the light entrance end surface 231 passes through the light-guiding member 230, emerges from the light exit end surface 232, and is then incident on the proximal end surface 61 of the tube 60.

The method of using the adapter 2 of Embodiment 2 is the same as that of Embodiment 1.

As in Embodiment 1, according to Embodiment 2, it is possible to make the distal end 65 (see FIG. 1) of the tube 60 emit light by using the tube 60 itself as a light-guiding path. Thus, it is possible to safely, easily, and accurately detect the position of the distal end 65 of the tube 60. There is no need to directly connect the connector 250 to the light source device 70, so that the occurrence of cross-infection can be prevented.

The adapter 2 includes the lens 237 for focusing light emitted from the light source 78 onto the light entrance end surface 231 of the light-guiding member 230.

Thus, light from the light source 78 can be made to efficiently enter the tube 60. This is advantageous in increasing the brightness (luminous flux) of the distal end 65 of the tube 60. Furthermore, the aperture of the lens 237 is larger than the diameter of the light entrance end surface 231. Alarger amount of light can be made to enter the light-guiding member 30 via the lens 237 that has a large area. This is advantageous in increasing the brightness (luminous flux) of the distal end 65 of the tube 60. However, the present invention is not limited to this configuration, and the adapter 2 need not include the lens 237. In this case, the light source 78 may include a lens and/or the light source device 70 may include a lens between the light source 78 and the light-guiding member 230 so that the light emitted from the light source 78 can be efficiently incident on the light entrance end surface 231 of the light-guiding member 230.

The adapter 2 includes the light-guiding member 230 that guides light emitted from the light source 78 to the proximal end surface 61 of the tube 60. Thus, light from the light source 78 can enter the tube 60 with high efficiency. This is advantageous in increasing the brightness (luminous flux) of the distal end 65 of the tube 60.

The light-guiding member 230 includes the light entrance end surface 231 provided with a convex curved surface. The convex curved surface refracts light from the light source 78 such that the light converges on the axis of the light-guiding member 230. This is advantageous in reducing light (leaked light) emerging from the side surface 233 of the light-guiding member 230. However, in the present invention, the light entrance end surface 231 need not have a convex curved surface. The light entrance end surface 231 may be a flat surface that is perpendicular to the axis of the adapter 2, or may be a concave curved surface.

The light-guiding member 230 includes the light exit end surface 232 that has an annular shape and opposes the proximal end surface 61, which has an annular shape, of the tube 60 in the axial direction. The annular light exit end surface 232 is advantageous in enabling light emerging from the light-guiding member 30 to be fully incident on the annular proximal end surface 61 of the tube 60.

The light exit end surface 232 has an annular flat surface. This is further advantageous in enabling light emerging from the light-guiding member 230 to fully enter the tube 60.

However, in the present invention, the shape of the light exit end surface 232 is not limited to the aforementioned shape. For example, the light exit end surface 232 may be a flat surface without the recess 234. Alternatively, the light exit end surface 232 may be a concave curved surface or a convex curved surface. For example, the cross-sectional shape of the light exit end surface 232 taken along a plane containing the axis of the adapter 2 may include two substantially circular arcs that are symmetrical to each other with respect to the axis, and the light exit end surface 232 may be an annular concave or convex curved surface formed according to a trajectory that is obtained when the two substantially circular arcs are rotated around the axis. Like the light exit end surface 32 of Embodiment 1, the light exit end surface 232 may have the function of a lens that refracts light emerging from the light-guiding member 230 such that the light converges on the annular proximal end surface 61.

It is preferable that at least a portion of the light exit end surface 232 of the light-guiding member 230 opposes the proximal end surface 61 of the tube 60 in the axial direction. When viewed along the axis of the adapter 2, a portion of the light exit end surface 232 may protrude outward or inward in the radial direction from the proximal end surface 61, or a portion of the proximal end surface 61 may protrude outward or inward in the radial direction from the light exit end surface 232. The inner and outer diameters of the light exit end surface 232 are preferably equal to, but may also be different from, the inner and outer diameters of the proximal end surface 61. The light exit end surface 232 is preferably coaxial, but may also be eccentric, with respect to the proximal end surface 61. The light exit end surface 232 preferably has a circular shape, but may also have a non-circular shape (e.g., an elliptical shape). The proximal end surface 61 of the tube 60 preferably has a circular shape, but may also be deformed into a non-circular shape (e.g., an elliptical shape).

The light-guiding member 230 need not increase in diameter near the light exit end surface 232. The light-guiding member 230 may have a constant diameter, or may decrease in diameter, near the light exit end surface 232.

When the second connecting portion 20 is connected to the connector 250, the light-guiding member 230 is inserted into the through hole 59 of the tubular member 52. In Embodiment 2, unlike Embodiment 1, the proximal end surface 61 of the tube 60 may be set back from the leading end of the tubular member 52. Therefore, irrespective of the inner diameter of the tube 60, the opening diameter (hereinafter referred to as the "leading end opening diameter") of the flow path (through hole 59) at the leading end of the tubular member 52 can be set to be equal to that of a male connector (hereinafter referred to as an "existing male connector", see FIGS. 4A and 4B of Patent Document 2, for example) provided at a proximal end of a conventional nasal tube used for tube feeding. The connector 250 (in particular, the connector portion 51) to which the adapter 2 is applied is easily configured to be interchangeable with an existing male connector.

However, the adapter 2 of Embodiment 2 can also be applied to the connector 50 (see FIGS. 2A and 2B) in which the tube 60 extends to the leading end of the tubular member 52, which has been described in Embodiment 1. In this case, the protruding length of the light-guiding member 230 into the second connecting tube 21 is shortened. When the second connecting portion 20 is connected to the connector 50, the light-guiding member 230 is not inserted into the through hole 59 of the tubular member 52, but the light exit end surface 232 opposes the proximal end surface 61 (or the leading end of the tubular member 52), as in the case of Embodiment 1.

The light-guiding member 230 may also protrude into the inner cavity of the first connecting tube 211 so that the light entrance end surface 231 is brought closer to the light source 78. A portion of the light-guiding member 230 that is located within the first connecting tube 211 may have a substantially truncated cone shape similar to that of the light-guiding member 30 of Embodiment 1. In this case, a larger amount of light can be made to enter the light-guiding member 230 via the light entrance end surface 231 having a large area.

Embodiment 2 is the same as Embodiment 1 except for the above-described differences. Descriptions of Embodiment 1 also apply to Embodiment 2.

### Embodiment 3

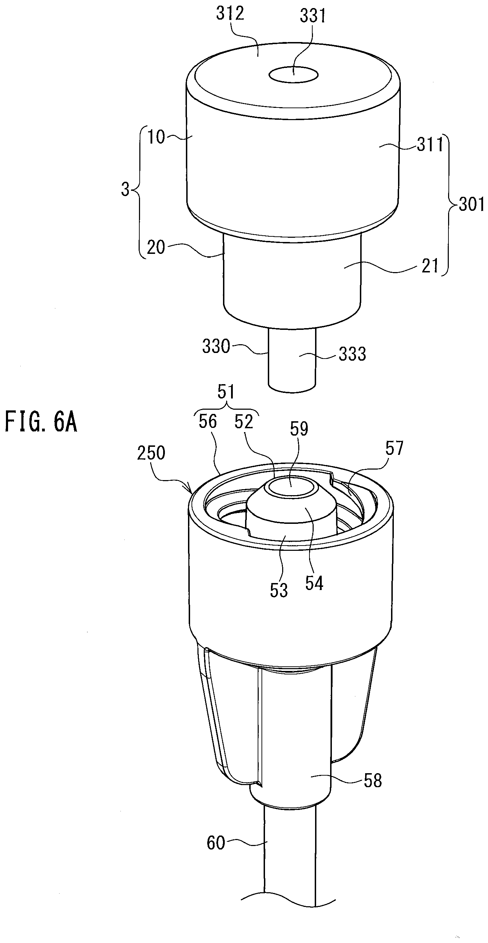

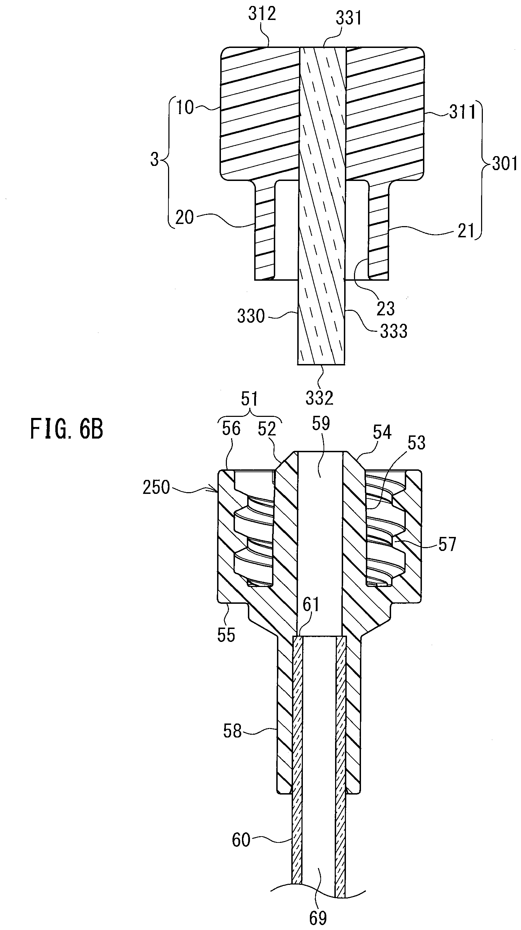

FIG. 6Ais a perspective view showing an adapter 3 according to Embodiment 3 of the present invention and the connector 250 provided at the proximal end of the tube 60. FIG. 6B is a cross-sectional view taken along a plane containing the axes (not shown) of the adapter 3 and the connector 250 in FIG. 6A. The adapter 3 of Embodiment 3 will be described focusing primarily on the differences from Embodiments 1 and 2.

The adapter 3 includes the first connecting portion 10 at one end and the second connecting portion 20 at the other end. The first connecting portion 10 and the second connecting portion 20 are arranged coaxially. The first connecting portion 10 includes a hollow cylindrical first connecting tube 311. An outer circumferential surface of the first connecting tube 311 is a cylindrical surface whose outer diameter is constant with respect to the axial direction, as in the case of the first connecting tube 11 of Embodiment 1. However, the outer circumferential surface of the first connecting tube 311 is not limited to this configuration, and may also be, for example, a tapered surface whose outer diameter gradually increases toward a leading end side or a base end side (second connecting portion 20 side) of the first connecting tube 311.

The adapter 3 further includes a light-guiding member 330. The light-guiding member 330 has an elongated rod shape (substantially cylindrical column shape) and extends coaxially with the adapter 3. The light-guiding member 330 includes a light entrance end surface 331 and a light exit end surface 332 that face opposite sides to each other in the axial direction, as well as a side surface 333 connecting the light entrance end surface 331 and the light exit end surface 332 to each other. Both the light entrance end surface 331 and the light exit end surface 332 are flat surfaces that are perpendicular to the axis of the adapter 3. The light entrance end surface 331 faces the leading end side of the first connecting portion 10 (the opposite side to the second connecting portion 20), and the light exit end surface 332 faces the opposite side to the first connecting portion 10. The side surface 333 is a cylindrical surface whose outer diameter is constant with respect to the axial direction. The light-guiding member 330 is fitted into the first connecting tube 311, with the side surface 333 being in surface contact with an inner circumferential surface of the first connecting tube 311 while leaving substantially no space therebetween. The light entrance end surface 331 is flush with a leading end surface 312 of the first connecting portion 10 (or the first connecting tube 311). The light-guiding member 330 protrudes further toward the opposite side to the first connecting portion 10 than the second connecting tube 21 of the second connecting portion 20 in the axial direction. The second connecting tube 21 surrounds the light-guiding member 330 while being spaced apart from the light-guiding member 330 in the radial direction.

The adapter 3 is composed of two components: the light-guiding member 330 and a portion (adapter main body 301) other than the light-guiding member 330. The adapter main body 301 is made of a material that has such a mechanical strength (stiffness) that it will not be substantially deformed by an external force. Materials that can be used for the adapter main body 301 of Embodiment 1 can be used as the material of the adapter main body 301 of Embodiment 3.

The light-guiding member 330 is made of a material (light-guiding material) having light-guiding properties so that light can enter the light-guiding member 330 from the light entrance end surface 331, pass through the light-guiding member 330, and emerge from the light exit end surface 332. Light-guiding materials that can be used for the light-guiding member 30 of Embodiment 1 can be used as the light-guiding material of the light-guiding member 330. In addition, the means for reducing leaked light from the side surface 33 of the light-guiding member 30, which has been described in Embodiment 1, can also be applied to the light-guiding member 330 as well.

There is no limitation on the method for integrating the light-guiding member 330 into the adapter main body 301. For example, a method in which the light-guiding member 330 and the adapter main body 301 are produced separately and then the light-guiding member 330 is fitted into the first connecting tube 311 of the adapter main body 301; a method in which one of the light-guiding member 330 and the adapter main body 301 is produced and then the other is integrated into the produced light-guiding member 330 or adapter main body 301 through double molding; and other methods can be used.

The connector 250 and the tube 60 are the same as the connector 250 and the tube 60 (see FIGS. 4A and 4B) of Embodiment 2.

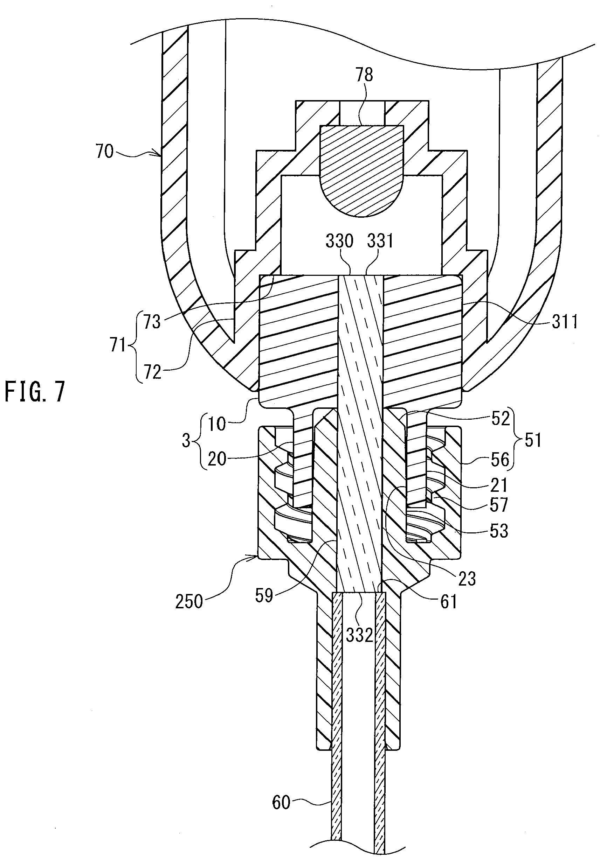

FIG. 7 is a cross-sectional view showing a state in which the connector 250 is connected to the light source device 70 via the adapter 3. The light source device 70 is the same as that of Embodiment 1. The first connecting portion 10 of the adapter 3 is inserted into the retaining portion 71 until the leading end of the first connecting tube 311 abuts against the step surface 73. The retaining cylinder 72 positions the first connecting portion 10 (or the light-guiding member 330) coaxially with the light source 78. The step surface 73 positions the first connecting portion 10 (or the light-guiding member 330) at a position located at a predetermined distance from the light source 78 in the axial direction. The light entrance end surface 331 of the light-guiding member 330 opposes the light source 78.

The second connecting portion 20 (or the second connecting tube 21) of the adapter 3 is connected to the connector 250 in a similar manner to that of Embodiment 1. The male tapered surface 53 of the tubular member 52 is taper-fitted to the female tapered surface 23 of the second connecting tube 21. As in Embodiment 2, the light-guiding member 330 is inserted into the through hole 59 of the tubular member 52. The light-guiding member 330 is positioned coaxially with the tube 60. The light exit end surface 332 of the light-guiding member 330 opposes the proximal end surface 61 of the tube 60 in the axial direction and is located in close proximity to, or preferably abuts against, the proximal end surface 61.

The light source 78 emits light toward the light entrance end surface 331 of the light-guiding member 330. The light emitted from the light source 78 is incident on the light entrance end surface 331, passes through the light-guiding member 330, emerges from the light exit end surface 332, and is then incident on the proximal end surface 61 of the tube 60.

The method of using the adapter 3 of Embodiment 3 is the same as that of Embodiment 1.

As in Embodiment 1, according to Embodiment 3, it is possible to make the distal end 65 (see FIG. 1) of the tube 60 emit light by using the tube 60 itself as a light-guiding path. Thus, it is possible to safely, easily, and accurately detect the position of the distal end 65 of the tube 60. There is no need to directly connect the connector 250 to the light source device 70, so that the occurrence of cross-infection can be prevented.

The adapter 3 includes the light-guiding member 330 that guides light emitted from the light source 78 to the proximal end surface 61 of the tube 60. Thus, light from the light source 78 can be made to efficiently enter the tube 60. This is advantageous in increasing the brightness (luminous flux) of the distal end 65 of the tube 60.

The light-guiding member 330 has a cylindrical column shape, with its side surface 333 being a cylindrical surface and its two end surfaces 331 and 332 being flat surfaces. Since the light-guiding member 330 has such a simple rod shape, it is easy to produce the light-guiding member 330. This is advantageous in reducing the cost of the adapter 3.

However, in the present invention, the shape of the light-guiding member 330 is not limited to the aforementioned shape.

For example, the light entrance end surface 331 may be a convex curved surface similar to the light entrance end surface 231 of Embodiment 2, or may be a concave curved surface. The light exit end surface 332 may be an annular flat surface having a recess 234 at the center, which is similar to the light exit end surface 232 of Embodiment 2. Alternatively, the light exit end surface 332 may be a concave or convex curved surface that is described as being applicable to the light exit end surface 232 in Embodiment 2. Like the light exit end surface 32 of Embodiment 1, the light exit end surface 332 may have the function of a lens that refracts light emerging from the light-guiding member 330 such that the light converges on the annular proximal end surface 61.

The side surface 333 of the light-guiding member 330 may have a shape other than a cylindrical surface. For example, the light-guiding member 330 may have, within the first connecting tube 311, a substantially truncated cone shape that gradually increases in diameter toward the light entrance end surface 331. In this case, like the light-guiding member 30 of Embodiment 1, the light entrance end surface 331 has a larger area than the light exit end surface 332. This is advantageous in increasing the brightness (luminous flux) of the distal end 65 of the tube 60. Alternatively, the diameter of the light-guiding member 330 may gradually increases, like the light-guiding member 230 (see FIG. 4B) of Embodiment 2, or may gradually decreases, near the light exit end surface 332.

The adapter 3 does not include the lens 237 (see FIGS. 4B and 5), which is included in the adapter 2 of Embodiment 2. Therefore, the number of components constituting the adapter 3 is reduced. The adapter 3 has a simple configuration and is easy to produce. This is advantageous in reducing the cost of the adapter 3.

Note that the light source 78 may include a lens and/or the light source device 70 may include a lens between the light source 78 and the light-guiding member 330 so that the light from the light source 78 can be efficiently incident on the light entrance end surface 331 of the light-guiding member 330.

In Embodiment 3, the light entrance end surface 331 is disposed at the same position as the leading end surface 312 of the first connecting portion 10 (the first connecting tube 311) with respect to the axial direction; however, the present invention is not limited to this configuration. The light entrance end surface 331 may protrude from, or may be set back from, the leading end of the first connecting tube 311.

The first connecting tube 311 need not be in surface contact with the side surface 333 of the light-guiding member 330. For example, a configuration may also be adopted in which the first connecting tube 311 has a hollow cylindrical shape like the first connecting tube 211 of Embodiment 2 and is spaced apart from the side surface 333 of the light-guiding member 330 in the radial direction.

It is preferable that at least a portion of the light exit end surface 332 of the light-guiding member 330 opposes the proximal end surface 61 of the tube 60 in the axial direction. When viewed along the axis of the adapter 3, a portion of the light exit end surface 332 may protrude outward in the radial direction from the proximal end surface 61, or a portion of the proximal end surface 61 may protrude outward in the radial direction from the light exit end surface 332. The outer diameter of the light exit end surface 332 is preferably equal to, but may also be different from, the outer diameter of the proximal end surface 61. The light exit end surface 332 is preferably coaxial, but may also be eccentric, with respect to the proximal end surface 61. The light exit end surface 332 preferably has a circular shape, but may also have a non-circular shape (e.g., an elliptical shape). The proximal end surface 61 of the tube 60 preferably has a circular shape, but may also be deformed into a non-circular shape (e.g., an elliptical shape).

As in Embodiment 2, when the second connecting portion 20 is connected to the connector 250, the light-guiding member 330 is inserted into the through hole 59 of the tubular member 52. Therefore, irrespective of the inner diameter of the tube 60, the opening diameter (leading end opening diameter) of the flow path (through hole 59) at the leading end of the tubular member 52 can be set to be equal to the opening diameter of a male connector (an existing male connector, see FIGS. 4A and 4B of Patent Document 2, for example) provided at a proximal end of a conventional nasal tube used for tube feeding. The connector 250 (in particular, the connector portion 51) to which the adapter 3 is applied is easily configured to be interchangeable with an existing male connector.

However, the adapter 3 of Embodiment 3 can also be applied to the connector 50 (see FIGS. 2A and 2B) in which the tube 60 extends to the leading end of the tubular member 52, which has been described in Embodiment 1. In this case, the protruding length of the light-guiding member 330 into the second connecting tube 21 is shortened. When the second connecting portion 20 is connected to the connector 50, the light-guiding member 330 is not inserted into the through hole 59 of the tubular member 52, but the light exit end surface 332 opposes the proximal end surface 61 (or the leading end of the tubular member 52), as in the case of Embodiment 1.

Embodiment 3 is the same as Embodiments 1 and 2, except for the above-described differences. Descriptions of Embodiments 1 and 2 that are applicable to Embodiment 3 also apply to Embodiment 3.

### Embodiment 4

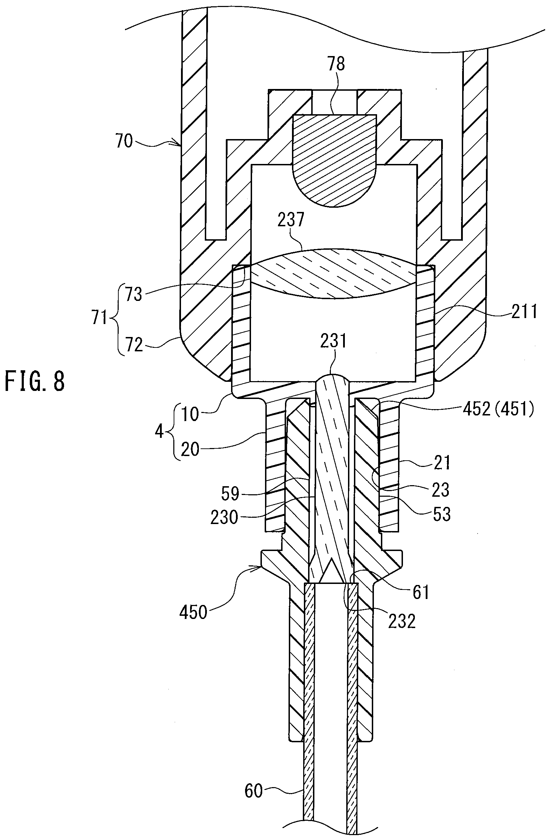

FIG. 8 is a cross-sectional view showing a state in which a connector 450 is connected to the light source device 70 via an adapter 4 according to Embodiment 4 of the present invention. The adapter 4 is substantially the same as the adapter 2 of Embodiment 2. The light source device 70 is the same as that of Embodiments 1 to 3. The connector 450 of Embodiment 4 does not include the outer cylinder 56, the female thread 57, and the flange 55, which are included in the connector 250 of Embodiment 2. A tubular member 452 of the connector 450 is fitted into the second connecting tube 21, and a frictional force therebetween connects the adapter 4 (in particular, the second connecting portion 20) to a connector potion 451 (in particular, the tubular member 452).

In Embodiment 2, the connector portion 51 is configured to be interchangeable with an existing male connector (see FIGS. 4A and 4B of Patent Document 2, for example) that is used for tube feeding. In contrast, in Embodiment 4, the connector portion 451 may also be a female connector that includes the tubular member 452 as a female member. In this case, any male member is removably inserted into an inner cavity (through hole 59) of the tubular member 452. An inner circumferential surface of the tubular member 452 may be provided with a female tapered surface whose inner diameter gradually increases toward a leading end of the tubular member 452. The female tapered surface can be configured to be taper-fitted to a male tapered surface provided on an outer circumferential surface of the male member. The tube 60 on which the connector 450 including the female connector 451 is provided may be used as a urethral catheter or a tracheal intubation catheter, for example.

Embodiment 4 is the same as Embodiment 2 except for the above-described differences. Descriptions of Embodiments 2 (and furthermore descriptions of Embodiment 1 that are also applicable to Embodiment 2) also apply to Embodiment 4. The adapter 3 of Embodiment 3 may also be connected to the connector 450.

### Embodiment 5

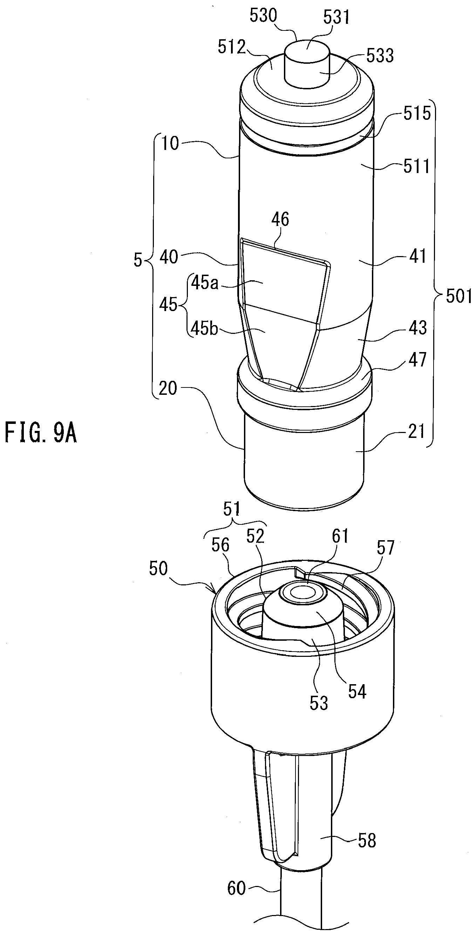

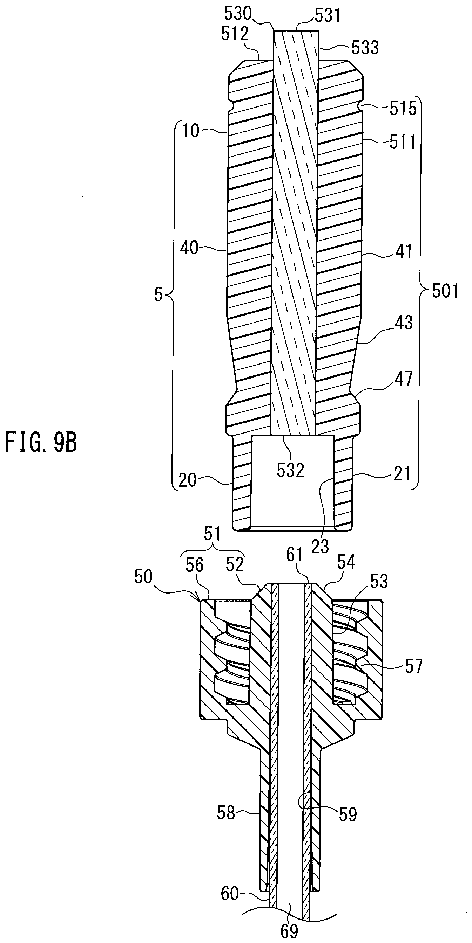

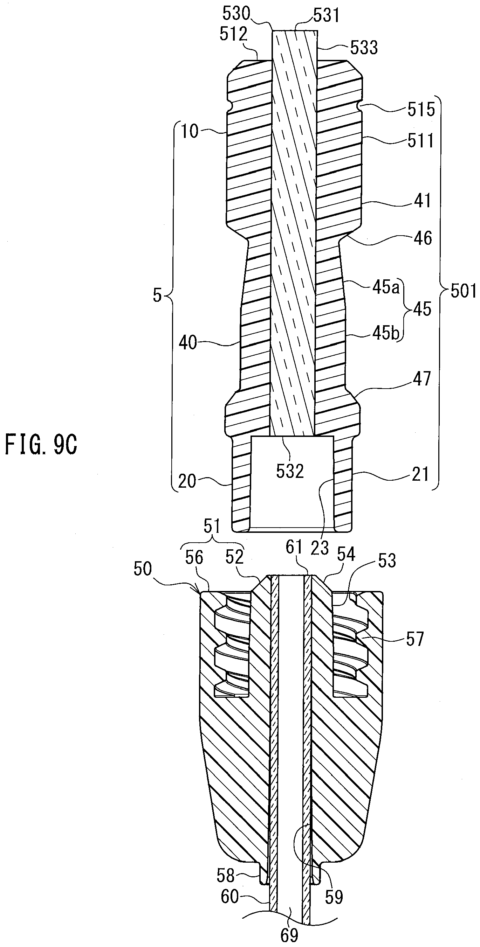

FIG. 9Ais a perspective view showing an adapter 5 according to Embodiment 5 of the present invention and the connector 50 provided at the proximal end of the tube 60. FIGS. 9B and 9C are cross-sectional views taken along a plane containing the axis (not shown) of the adapter 5 in FIG. 9A The cross section shown in FIG. 9B and the cross section shown in FIG. 9C are orthogonal to each other, where the axis of the adapter 5 is the line of intersection. The adapter 5 of Embodiment 5 will be described focusing primarily on the differences from Embodiments 1 and 3.

The adapter 5 includes the first connecting portion 10 at one end and the second connecting portion 20 at the other end. The first connecting portion 10 and the second connecting portion 20 are arranged coaxially. The first connecting portion 10 includes a hollow cylindrical first connecting tube 511. An outer circumferential surface of the first connecting tube 511 is a cylindrical surface whose outer diameter is constant with respect to the axial direction, as in the case of the first connecting tube 11 of Embodiment 1. However, the outer circumferential surface of the first connecting tube 511 is not limited to this configuration, and may also be, for example, a tapered surface whose outer diameter gradually increases toward a leading end side or a base end side (second connecting portion 20 side) of the first connecting tube 511. An annular recess (annular groove) 515 is formed on the outer circumferential surface of the first connecting tube 511, the annular recess 515 extending continuously over the entire circumference in the circumferential direction.

The first connecting portion 10 and the second connecting portion 20 are separated from each other in the axial direction, and an intermediate portion 40 is provided therebetween. The intermediate portion 40 includes a hollow cylindrical intermediate tube 41. The first connecting tube 511 and the intermediate tube 41 have a continuous through hole extending along the axis of the adapter 5. An outer circumferential surface of the intermediate portion 40 (or the intermediate tube 41) is, but not limited to, a cylindrical surface having substantially the same diameter as the first connecting tube 511. However, a tapered surface 43 and a pair of holding surfaces 45 are formed on the outer circumferential surface of the intermediate portion 40 by cutting off (or shaving off) portions of the cylindrical surface of the intermediate portion 40 (see FIG. 9C). The tapered surface 43 is a conical surface whose outer diameter gradually decreases toward the second connecting portion 20. The pair of holding surfaces 45 are symmetrical to each other with respect to the axis of the adapter 5. Each holding surface 45 is constituted by a first flat surface 45a located on the first connecting portion 10 side and a second flat surface 45b located on the second connecting portion 20 side. The pair of first flat surfaces 45a are inclined such that the distance therebetween gradually decreases toward the first connecting portion 10. The pair of second flat surfaces 45b are parallel to each other. A rising surface (first rising surface) 46 is formed adjoining the first flat surface 45a on the first connecting portion 10 side. The rising surface 46 is inclined radially outward toward the first connecting portion 10. A rising surface (second rising surface) 47 is formed adjoining the tapered surface 43 and the second flat surface 45b on the second connecting portion 20 side. The rising surface 47 is inclined radially outward toward the second connecting portion 20. The rising surface 47 is continuous over the entire circumference of the intermediate portion 40 in the circumferential direction and has an annular shape.

The adapter 5 further includes a light-guiding member 530. The light-guiding member 530 has an elongated rod shape (substantially cylindrical column shape) and extends coaxially with the adapter 5. The light-guiding member 530 includes a light entrance end surface 531 and a light exit end surface 532 that face opposite sides to each other in the axial direction, as well as a side surface 533 connecting the light entrance end surface 531 and the light exit end surface 532 to each other. Both the light entrance end surface 531 and the light exit end surface 532 are flat surfaces that are perpendicular to the axis of the adapter 5. The light entrance end surface 531 faces the opposite side to the second connecting portion 20, and is, in Embodiment 5, a flat surface that is perpendicular to the axis of the adapter 5. Unlike Embodiment 3, the light-guiding member 530 protrudes from a leading end surface 512 of the first connecting portion 10 (or the first connecting tube 511) in the axial direction. The light exit end surface 532 faces the opposite side to the first connecting portion 10. Unlike Embodiment 3, the light-guiding member 530 does not protrude into the second connecting tube 21. The side surface 533 is a cylindrical surface whose outer diameter is constant with respect to the axial direction. The light-guiding member 530 is fitted into an adapter main body 501, with the side surface 533 (excluding a portion protruding from the leading end surface 512) being in surface contact with an inner circumferential surface of a through hole of the adapter main body 501 while leaving substantially no space therebetween.

The adapter 5 is composed of two components: the light-guiding member 530 and a portion (adapter main body 501) other than the light-guiding member 530. The adapter main body 501 is made of a material that has such a mechanical strength (stiffness) that it will not be substantially deformed by an external force. Materials that can be used for the adapter main body 101 of Embodiment 1 can be used as the material of the adapter main body 501 of Embodiment 5.

The light-guiding member 530 is made of a material (light-guiding material) having light-guiding properties so that light can enter the light-guiding member 530 from the light entrance end surface 531, pass through the light-guiding member 530, and emerge from the light exit end surface 532. Light-guiding materials that can be used for the light-guiding member 30 of Embodiment 1 can be used as the light-guiding material of the light-guiding member 530. In addition, the means for reducing leaked light from the side surface 33 of the light-guiding member 30, which has been described in Embodiment 1, can also be applied to the light-guiding member 530 as well.

There is no limitation on the method for integrating the light-guiding member 530 into the adapter main body 501. For example, a method in which the light-guiding member 530 and the adapter main body 501 are produced separately and then the light-guiding member 530 is fitted into the through hole of the adapter main body 501; a method in which one of the light-guiding member 530 and the adapter main body 501 is produced and then the other is integrated into the produced light-guiding member 530 or adapter main body 501 through double molding; and other methods can be used.

The connector 50 and the tube 60 are the same as the connector 50 and the tube 60 (see FIGS. 2A and 2B) of Embodiment 1.

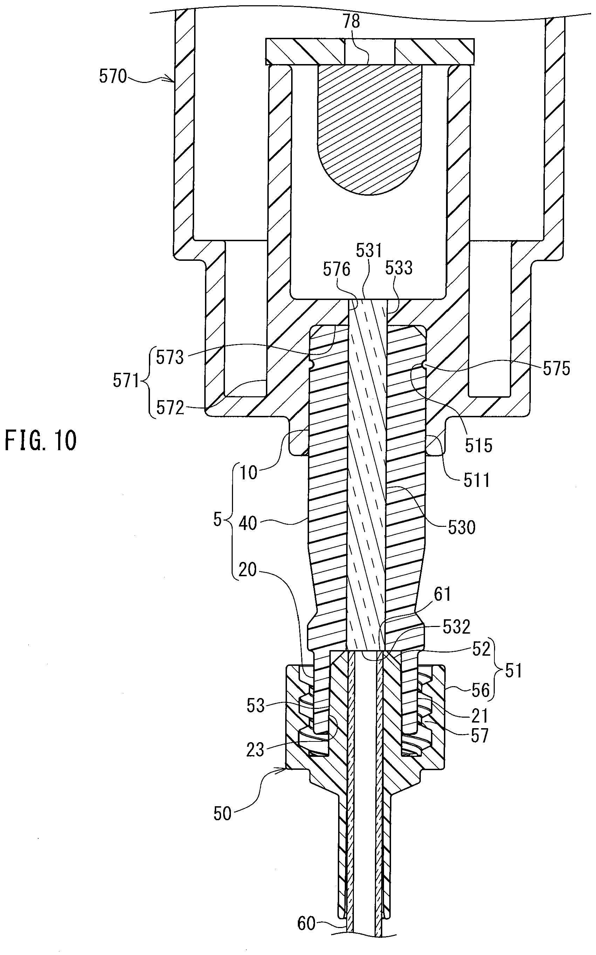

FIG. 10 is a cross-sectional view showing a state in which the connector 50 is connected to a light source device 570 via the adapter 5.

The light source device 570 includes a retaining portion 571 in which the first connecting portion 10 (first connecting tube 511) is accommodated and retained, and a light source 78 disposed at a position farther inward than the retaining portion 571. The retaining portion 571 includes a retaining cylinder 572 and a bottom plate 573. An inner circumferential surface of the retaining cylinder 572 is a cylindrical surface having substantially the same diameter as the first connecting tube 511. Four protrusions 575 (only two of the protrusions 575 can be seen in FIG. 10) protrude inward in the radial direction from the inner circumferential surface of the retaining cylinder 572. The four protrusions 575 are arranged at equal angular intervals in the circumferential direction. The bottom plate 573 is formed at a position located at a predetermined distance from an opening end of the retaining cylinder 572 and protrudes inward in the radial direction from the inner circumferential surface of the retaining cylinder 572. The bottom plate 573 has a through hole 576 at the center, the through hole 576 penetrating the bottom plate 573 in the axial direction. The light source 78 is disposed coaxially with the through hole 576 (or the retaining portion 571). The first connecting portion 10 of the adapter 5 is inserted into the retaining portion 571 until the leading end surface 512 of the first connecting tube 511 abuts against, or comes into close proximity to, the bottom plate 573. The protrusions 575 of the retaining cylinder 572 are fitted into the annular recess 515 of the first connecting portion 10 (or the first connecting tube 511), and thus, the adapter 5 is locked to the light source device 570. The protrusions 575 and/or the bottom plate 573 positions the first connecting portion 10 (or the light-guiding member 530) at a position located at a predetermined distance from the light source 78 in the axial direction. The light-guiding member 530 protruding from the leading end surface 512 of the first connecting portion 10 (the first connecting tube 511) is fitted into the through hole 576 of the bottom plate 573. An inner circumferential surface of the through hole 576, which defines the through hole 576, positions the light-guiding member 530 coaxially with the light source 78. The light entrance end surface 531 of the light-guiding member 530 opposes the light source 78.

The second connecting portion 20 (or the second connecting tube 21) of the adapter 5 is connected to the connector 50 in a similar manner to that of Embodiment 1. The male tapered surface 53 of the tubular member 52 is taper-fitted to the female tapered surface 23 of the second connecting tube 21. The light exit end surface 532 of the light-guiding member 530 opposes the proximal end surface 61 of the tube 60 in the axial direction and is located in close proximity to, or preferably abuts against, the proximal end surface 61.

The light source 78 emits light toward the light entrance end surface 531 of the light-guiding member 530. The light emitted from the light source 78 is incident on the light entrance end surface 531, passes through the light-guiding member 530, emerges from the light exit end surface 532, and is then incident on the proximal end surface 61 of the tube 60.

The method of using the adapter 5 of Embodiment 5 is the same as that of Embodiment 1.

As in Embodiment 1, according to Embodiment 5, it is possible to make the distal end 65 (see FIG. 1) of the tube 60 emit light by using the tube 60 itself as a light-guiding path. Thus, it is possible to safely, easily, and accurately detect the position of the distal end 65 of the tube 60. There is no need to directly connect the connector 50 to the light source device 70, so that the occurrence of cross-infection can be prevented.

The adapter 5 includes the light-guiding member 530 that guides light emitted from the light source 78 to the proximal end surface 61 of the tube 60. Thus, light from the light source 78 can be made to efficiently enter the tube 60. This is advantageous in increasing the brightness (luminous flux) of the distal end 65 of the tube 60.

The side surface 533 of the light-guiding member 530 protrudes from the leading end surface 512 of the first connecting portion 10 (the first connecting tube 511). The protruding side surface 533 of the light-guiding member 530 opposes the inner circumferential surface of the through hole 576, which defines the through hole 576, of the light source device 570 in the radial direction. Thus, the axis of the light-guiding member 530 is aligned with the axis of the light source 78 (see FIG. 10). Since the axial alignment is performed using the light-guiding member 530, which includes the light entrance end surface 531 on which light from the light source 78 is incident, eccentricity of the light-guiding member 530 (or the light entrance end surface 531) with respect to the light source 78 can be reduced. This can increase the amount of light that enters the light-guiding member 530 and is therefore advantageous in increasing the brightness (luminous flux) of the distal end 65 of the tube 60.

In Embodiment 5, the side surface 533 of the light-guiding member 530 protruding from the leading end surface 512 is a cylindrical surface, and the inner circumferential surface of the through hole 576 is also a cylindrical surface. However, the present invention is not limited to this configuration. For example, the side surface 533 of the light-guiding member 530 protruding from the leading end surface 512 may be a tapered surface (conical surface) whose outer diameter gradually decreases toward the light entrance end surface 531. Additionally or alternatively, the inner circumferential surface of the through hole 576 may be a tapered surface (conical surface) whose inner diameter gradually increases as the distance from the light source 78 increases. In these cases, the light-guiding member 530 and the inner circumferential surface of the through hole 576 are fitted to each other as a result of the cylindrical surface and the tapered surface being fitted to each other or the tapered surface and the tapered surface being fitted (so-called taper-fitted) to each other. This is advantageous in reducing the eccentricity of the light-guiding member 530 (or the light entrance end surface 531) with respect to the light source 78.

In Embodiment 5, the light-guiding member 530 need not protrude from the leading end surface 512 of the first connecting portion 10. In this case, the axis of the first connecting portion 10 (or the light-guiding member 530) may be aligned with the axis of the light source 78 in a similar manner to that of Embodiment 1.

When the adapter 5 (the first connecting portion 10) is connected to the light source device 570, the protrusions 575 of the light source device 570 are engaged with the annular recess 515 of the adapter 5. This is advantageous in preventing the adapter 5 from dislodging from the light source device 570 when a pulling force is applied between the adapter 5 and the light source device 570, and in maintaining a stable connection between the adapter 5 and the light source device 570. The adapter 5 is prevented from moving relative to (in particular, moving away from) the light source device 570 in the axial direction, and the adapter 5 is positioned and retained in a predetermined position relative to the light source device 570 in the axial direction. A user can easily confirm that the adapter 5 (the first connecting portion 10) is properly connected to the light source device 570, by perceiving a click feel (or a click sound) that is generated when the aforementioned engagement is established.

Since the recess 515 extends continuously over the entire circumference of the first connecting portion 10 and has an annular shape, when connecting the adapter 5 to the light source device 570, it is not necessary to align the adapter 5 with the light source device 570 in a rotating direction. Thus, the adapter 5 can be connected to the light source device 570 by simply inserting the first connecting portion 10 of the adapter 5 into the light source device 570. Also, the light source device 570 can be rotated relative to the adapter 5 after the adapter 5 has been connected to the light source device 570. Thus, the position of the light source device 570 in the rotating direction can be adjusted without twisting the tube 60.

In Embodiment 5, mutually engageable engagement structures provided on the adapter 5 and the light source device 570 are the protrusions 575 and the annular recess 515, respectively. However, the engagement structures of the present invention are not limited to this configuration. In general, the engagement structures respectively provided on the adapter and the light source device may be a protrusion and a recess that are mutually engageable, or a protrusion and a protrusion that are mutually engageable. When the engagement structures are constituted by a protrusion and a recess, the adapter may be provided with either the protrusion or the recess. The protrusion and/or the recess may extend annularly over the entire circumference, or may be divided into sections in the circumferential direction (i.e., non-annular). The number of non-annular protrusions and/or recesses may be one or more. A configuration may also be adopted in which the protrusion and/or the recess is supported by an elastically deformable member (e.g., a compression coil spring or a bendable arm) and can be moved (e.g., moved in the radial direction).

In Embodiment 5, the adapter 5 and the light source device 570 need not be provided with the mutually engageable engagement structures (the protrusions 575 and the annular recess 515). The adapter and the light source device of Embodiments 1 to 4 may be provided with mutually engageable engagement structures similar to those of Embodiment 5.

The adapter 5 includes the intermediate portion 40 between the first connecting portion 10 and the second connecting tube 20. As can be seen from a comparison between FIG. 10 and FIG. 3, in Embodiment 5, when the connector 50 is connected to the light source device 570 via the adapter 5, the connector 50 and the light source device 570 are spaced apart from each other, and the intermediate portion 40 is exposed to the outside. The user can hold the intermediate portion 40 when handling the adapter 5. As a result, the user is less likely to touch the first connecting portion 10 and the second connecting portion 20. This is advantageous in reducing the likelihood of the first connecting portion 10 and the second connecting portion 20 being contaminated by the fingers of the user.

When the user holds the intermediate portion 40 with his/her fingers, the user can hold the holding surfaces 45 with his/her fingers. This makes it possible to stably hold the adapter 5 and makes it easier to apply a force acting in the axial direction and/or a rotational force around the axis to the adapter 5. In Embodiment 5, the two holding surfaces 45 axially symmetrical to each other are provided on the intermediate portion 40. However, a holding surface of the present invention is not limited to this configuration. The number of holding surfaces is not limited to two, and may be more than two. In general, it is preferable that an even number of holding surfaces are arranged so as to form a regular polygon (e.g., a regular quadrangle, a regular hexagon, or the like) in a cross section taken along a plane that is perpendicular to the axis of the adapter 5.

It is preferable that the holding surfaces 45 are each constituted by a flat surface. However, the holding surface is not limited to an exact flat surface. The holding surface may be a convex curved surface or a concave curved surface, for example. The holding surface may be provided with a plurality of ribs, a plurality of projections, or the like for slip prevention. It is preferable that the holding surface is configured to be distinguishable from the outer circumferential surface (cylindrical surface or tapered surface) of the intermediate portion 40.

In Embodiment 5, each holding surface 45 is constituted by the two flat surfaces 45a and 45b having different inclinations. However, the holding surface of the present invention is not limited to this configuration. Each holding surface may consist of a single surface, or three or more surfaces. In a cross section containing the axis of the adapter 5, a surface constituting the holding surface may be parallel to the axis of the adapter 5 or may be inclined with respect to the axis of the adapter 5.

In Embodiment 5, the holding surfaces 45 are formed by cutting off portions of the outer circumferential surface of the intermediate portion 40. However, the holding surface of the present invention is not limited to this configuration. For example, the holding surface may be provided protruding outward in the radial direction from the outer circumferential surface of the intermediate portion 40. Alternatively, like the pair of plates (see FIG. 9A) protruding from the base tube 58 of the connector 50, a pair of plates that are parallel to the axis of the adapter 5 may be provided protruding outward in the radial direction from the outer circumferential surface of the intermediate portion 40, and surfaces of those plates may be used as holding surfaces.

The rising surfaces 46 and 47 adjoining the holding surfaces 45 function as a "holding position restricting structure" that restricts the positions at which the intermediate portion 40 is held by the fingers with respect to the axial direction so that the fingers holding the holding surfaces 45 do not protrude from the holding surfaces 45 in the axial direction. The rising surfaces 46 and 47 serving as the holding position restricting structure make it easier to apply a force acting in the axial direction to the adapter 5 when connecting the adapter 5 to the connector 50 and the light source device 570. Also, the rising surfaces 46 and 47 reduce the likelihood of the user touching the first connecting portion 10 and the second connecting portion 20 when handling the adapter 5, and thus reduce the likelihood of the first connecting portion 10 and the second connecting portion 20 being contaminated by the fingers of the user.

The inclinations of the rising surfaces 46 and 47 with respect to the axis of the adapter 5 in a cross section containing the axis of the adapter 5 may be any values. The rising surfaces 46 and 47 preferably extend along the circumferential direction, and may be continuous over the entire circumference of the intermediate portion 40 in the circumferential direction and have an annular shape. The rising surfaces 46 and 47 preferably adjoin regions (the holding surfaces 45) to be held by the fingers in the axial direction. In Embodiment 5, a configuration may also be adopted in which the holding surfaces 45 are omitted, and the rising surfaces 46 and 47 are provided on the outer circumferential surface of the intermediate portion 40 while opposing each other and being spaced apart from each other in the axial direction. In this case as well, the rising surfaces 46 and 47 can function as the holding position restricting structure.

In Embodiment 5, the adapter 5 need not be provided with one or both of the rising surfaces 46 and 47. Moreover, the adapter 5 need not be provided with the tapered surface 43.

The intermediate portion 40 of Embodiment 5 may also be applied to the adapters 1 to 4 of Embodiments 1 to 4. In this case, the intermediate portions 40 of the adapters 1 to 4 may be provided with the holding surfaces and/or the rising surfaces of Embodiment 5.

The light-guiding member 530 has a cylindrical column shape, with its side surface 533 being a cylindrical surface and its two end surfaces 531 and 532 being flat surfaces. Since the light-guiding member 530 has such a simple rod shape, it is easy to produce the light-guiding member 530. This is advantageous in reducing the cost of the adapter 5.

However, in the present invention, the shape of the light-guiding member 530 is not limited to the aforementioned shape.

For example, the light entrance end surface 531 may be a convex curved surface similar to the light entrance end surface 231 of Embodiment 2, or may be a concave curved surface. The light exit end surface 532 may be an annular flat surface having a recess 234 at the center, which is similar to the light exit end surface 232 of Embodiment 2. Alternatively, the light exit end surface 532 may be a concave or convex curved surface that is described as being applicable to the light exit end surface 232 in Embodiment 2. Like the light exit end surface 32 of Embodiment 1, the light exit end surface 532 may have the function of a lens that refracts light emerging from the light-guiding member 530 such that the light converges on the annular proximal end surface 61.

The side surface 533 of the light-guiding member 530 may also have a shape other than a cylindrical surface. For example, the light-guiding member 530 may have, within the adapter main body 501 (or the first connecting tube 511), a substantially truncated cone shape that gradually increases in diameter toward the light entrance end surface 531. In this case, like the light-guiding member 30 of Embodiment 1, the light entrance end surface 531 has a larger area than the light exit end surface 532. This is advantageous in increasing the brightness (luminous flux) of the distal end 65 of the tube 60. However, even in this case, the side surface 533 of the light-guiding member 530 protruding from the leading end surface 512 is preferably a cylindrical surface, or a tapered surface (conical surface) whose outer diameter gradually decreases toward the light entrance end surface 531, as described above.

The cross-sectional shape of the light-guiding member 530 taken along a plane that is perpendicular to the axial direction of the light-guiding member 530 is not limited to a circle. The cross-sectional shape of the light-guiding member 530 may be any shape, such as an ellipse, a polygon (e.g., a triangle, a quadrangle, or a hexagon; a corner of the polygon may also be chamfered into a circular arc, a straight line, or the like), or a shape obtained by connecting both ends of a circular arc (not necessarily an exact circular arc) with a straight line. The cross-sectional shape of the light-guiding member 530 need not be constant with respect to the axial direction of the light-guiding member 530 and may be varied.

The adapter 5 does not include the lens 237 (see FIGS. 4B and 5), which is included in the adapter 2 of Embodiment 2. Therefore, the number of components constituting the adapter 5 is reduced. The adapter 5 has a simple configuration and is easy to produce. This is advantageous in reducing the cost of the adapter 5.

Note that the light source 78 may include a lens and/or the light source device 570 may include a lens between the light source 78 and the light-guiding member 530 so that the light from the light source 78 can be efficiently incident on the light entrance end surface 531 of the light-guiding member 530.

The first connecting tube 511 and the intermediate tube 41 need not be in surface contact with the side surface 533 of the light-guiding member 530. For example, the first connecting tube 511 and/or the intermediate tube 41 may be spaced apart from the side surface 533 of the light-guiding member 530 in the radial direction.

It is preferable that at least a portion of the light exit end surface 532 of the light-guiding member 530 opposes the proximal end surface 61 of the tube 60 in the axial direction. When viewed along the axis of the adapter 5, a portion of the light exit end surface 532 may protrude outward in the radial direction from the proximal end surface 61, or a portion of the proximal end surface 61 may protrude outward in the radial direction from the light exit end surface 532. The outer diameter of the light exit end surface 532 is preferably equal to, but may also be different from, the outer diameter of the proximal end surface 61. The light exit end surface 532 is preferably coaxial, but may also be eccentric, with respect to the proximal end surface 61. The light exit end surface 532 preferably has a circular shape, but may also have a non-circular shape (e.g., an elliptical shape). The proximal end surface 61 of the tube 60 preferably has a circular shape, but may also be deformed into a non-circular shape (e.g., an ellipse, a polygon (e.g., a triangle, a quadrangle, or a hexagon; a corner of the polygon may be curved into a circular arc shape).

As in Embodiment 1, when the second connecting portion 20 is connected to the connector 50, the light-guiding member 530 is not inserted into the tubular member 52. The light-guiding member 530 may oppose the leading end of the tubular member 52 in the axial direction. This is advantageous in simplifying the configuration of the adapter 5. Thus, it is possible to easily optically couple the light-guiding member 530 to the tube 60 using a simple and inexpensive adapter 5.

However, the adapter 5 of Embodiment 5 may also be configured to be connectable to the connector 250 of Embodiments 2 and 3 or the connector 450 of Embodiment 4. In this case, the light-guiding member 530 may be configured to be inserted into the through hole 59 of the tubular member 52 of the connector (250 or 450). The light exit end surface 532 and its neighboring portion may be configured similarly to the light-guiding members of Embodiments 2 to 4.

Embodiment 5 is the same as Embodiments 1 and 4, except for the above-described differences. Descriptions of Embodiments 1 and 4 that are applicable to Embodiment 5 also apply to Embodiment 5.

It should be understood that Embodiments 1 to 5 above are given by way of example only. The present invention is not limited to Embodiments 1 to 5 above, and modifications can be made thereto as appropriate.

The connection structure between the adapter of the present invention and a light source device is not limited to those described in Embodiments 1 to 5 above. In Embodiments 1 to 5 above, the retaining cylinder (72, 572) of the light source device (70, 570) is configured such that the first connecting tube (11, 211, 311, 511) is fitted into the retaining cylinder (72, 572); however, the present invention is not limited to this configuration. The light source device may not include the retaining cylinder (72, 572) into which the first connecting tube (11, 211, 311, 511) is fitted. In this case, the adapter can be connected to the light source device in a state in which the leading end of the first connecting tube (11, 211, 311, 511) abuts against a flat surface that is provided on the light source device and is equivalent to the step surface 73 or the bottom plate 573. The flat surface positions the first connecting portion 10 (or the light-guiding member (30, 230, 330, 530)) at a position located at a predetermined distance from the light source 78 in the axial direction. Alight source device that does not include the retaining cylinder (72, 572) has a simple structure and is easy to produce. There is no limitation on the method for maintaining the state in which the adapter is connected to the light source device, and, for example, any method such as a method in which an elastic band is used or a method that uses magnetic attraction force can be selected.

The connection structure between the adapter of the present invention and the connector is not limited to those described in Embodiments 1 to 5 above. In Embodiments 1 to 5 above, the female tapered surface 23 of the second connecting portion 20 of the adapter is taper-fitted to the male tapered surface 53 of the connector; however, the second connecting portion 20 may have a fitting structure that can be fitted to the connector using a fitting method other than taper-fitting. For example, the inner circumferential surface of the second connecting tube 21 may have a female tapered surface with a different taper angle than the male tapered surface 53 of the connector, or may have a cylindrical surface. Alternatively, the outer circumferential surface of the tubular member (52, 452) of the connector may have a cylindrical surface. Even in such cases, the inner circumferential surface of the second connecting tube 21 can be fitted to the outer circumferential surface of the tubular member (52, 452) using a fitting method other than taper-fitting. Moreover, the second connecting portion 20 of the adapter may be configured to be fitted to a portion other than the outer circumferential surface of the tubular member (52, 452) of the connector.

The light-guiding member (30, 230, 330, 530) may be formed by a single optical fiber or a plurality of optical fibers (an optical fiber bundle) extending substantially along the axis of the adapter. In this case, light from the light source 78 passes through the single optical fiber or the plurality of optical fibers before being incident on the proximal end surface 61 of the tube 60.

In the present invention, the wording "the light-guiding tube is drawn out from the base tube of the connector toward the side opposite to the tubular member" means that, as seen from the outside, the tube 60 extends from the base tube 58 and does not mean that the proximal end surface 61 of the tube 60 is located within the base tube 58. That is to say, in the present invention, the insertion depth of the tube 60 into the connector can be set to any depth, and, for example, in Embodiments 2 to 4, the tube 60 may be inserted deeply into the connector 250, 350, or 450 such that the proximal end surface 61 is located in the tubular member 52 or 452.

The inner diameter of the through hole 59 of the tubular member of the connector to which the adapter of the present invention is connected may be constant, or may vary, with respect to the axial direction of the connector. The diameter (inner diameter and/or outer diameter) of the tube 60 may be constant, or may vary, within the connector.

### Industrial Applicability

The present invention can be widely used as an adapter for connecting a connector provided at a proximal end of a medical tube to be inserted into a patient to a light source device.

### Description of Reference Numerals

- 1, 2, 3, 4, 5: Adapter
- 10: First connecting portion
- 11, 211, 311, 511: First connecting tube
- 312, 512: Leading end surface of first connecting portion (first connecting tube)
- 20: Second connecting portion
- 21: Second connecting tube (Fitting structure)
- 30, 230, 330, 530: Light-guiding member
- 31, 231, 331, 531: Light entrance end surface
- 32, 232, 332, 532: Light exit end surface
- 33, 533: Side surface of light-guiding member
- 40: Intermediate portion
- 45: Holding surface
- 46, 47: Rising surface (Holding position restricting structure)
- 50,250,450: Connector
- 52, 452: Tubular member
- 56: Outer cylinder
- 57: Female thread
- 58: Base tube
- 60: Tube (Light-guiding tube)
- 61: Proximal end surface of tube
- 65: Distal end of tube
- 69: Flow path of tube
- 70: Light source device
- 237: Lens
- 515: Annular recess (Engagement structure)
- 575: Protrusion (Engagement structure)

## Claims

1. An adapter for connecting a connector provided at a proximal end of a flexible hollow light-guiding tube to a light source device,
the connector including a hollow cylindrical tubular member and a hollow cylindrical base tube that is disposed coaxially with the tubular member, with the light-guiding tube drawn out from the base tube toward a side opposite to the tubular member,
wherein the adapter comprises:
a first connecting portion that can be connected to and disconnected from the light source device;
a second connecting portion that can be connected to and disconnected from the connector; and
a light-guiding member that guides light emitted from the light source device to a proximal end surface of the light-guiding tube.

2. The adapter according to claim 1,
wherein the light-guiding member includes a light entrance end surface on which light from the light source device is incident, and the light entrance end surface is provided with a convex curved surface.

3. The adapter according to claim 1 or 2,
wherein the light-guiding member includes an annular light exit end surface from which light is emitted toward the proximal end surface of the light-guiding tube.

4. The adapter according to any one of claims 1 to 3,
wherein the light-guiding member includes a light exit end surface from which light is emitted toward the proximal end surface of the light-guiding tube, and
the light exit end surface has the function of a lens for focusing light toward the proximal end surface of the light-guiding tube.

5. The adapter according to any one of claims 1 to 4,
wherein the light-guiding member includes a light entrance end surface on which light from the light source device is incident and a light exit end surface from which light is emitted toward the proximal end surface of the light-guiding tube, and
the light entrance end surface has a larger area than the light exit end surface.

6. The adapter according to any one of claims 1 to 4,
wherein the light-guiding member includes a light entrance end surface on which light from the light source device is incident and a light exit end surface from which light is emitted toward the proximal end surface of the light-guiding tube, and
the light-guiding member has a constant outer diameter between the light entrance end surface and the light exit end surface.

7. The adapter according to any one of claims 1 to 6,
wherein, when the second connecting portion is connected to the connector, the light-guiding member opposes the tubular member in an axial direction.

8. The adapter according to any one of claims 1 to 6,
wherein, when the second connecting portion is connected to the connector, the light-guiding member is inserted into the tubular member.

9. The adapter according to claim 8, further comprising a lens for focusing light emitted from the light source device toward a light entrance end surface of the light-guiding member.

10. The adapter according to any one of claims 1 to 8,
wherein a side surface of the light-guiding member protrudes from a leading end surface of the first connecting portion in the axial direction.

11. The adapter according to any one of claims 1 to 10, further comprising an engagement structure that is engaged with the light source device when the first connecting portion is connected to the light source device.

12. The adapter according to any one of claims 1 to 11, further comprising an intermediate portion between the first connecting portion and the second connecting portion.

13. The adapter according to claim 12,
wherein the intermediate portion is provided with a holding surface.

14. The adapter according to claim 12 or 13,
wherein the intermediate portion is provided with a rising surface that protrudes outward in a radial direction.

15. The adapter according to any one of claims 1 to 14,
wherein the second connecting portion includes a fitting structure that can be fitted to the connector.

16. The adapter according to any one of claims 1 to 15,
wherein the connector further includes an outer cylinder surrounding the tubular member and a female thread provided on an inner circumferential surface of the outer cylinder that opposes the tubular member.
